(19) Europäisches Patentamt
European Patent Office
Office européen des brevets

(11) **EP 2 944 350 B1**

(12) **EUROPÄISCHE PATENTSCHRIFT**

(45) Veröffentlichungstag und Bekanntmachung des Hinweises auf die Patenterteilung:
**18.12.2019   Patentblatt 2019/51**

(51) Int Cl.:
*A61N 1/32* (2006.01)          *A61N 1/40* (2006.01)

(21) Anmeldenummer: **15166253.3**

(22) Anmeldetag: **20.06.2013**

(54) **THERAPIEVORRICHTUNG**

THERAPY DEVICE

DISPOSITIF DE THÉRAPIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.06.2012   DE 202012006092 U**
          **15.11.2012   DE 202012010985 U**
          **07.03.2013   DE 102013102275**

(43) Veröffentlichungstag der Anmeldung:
**18.11.2015   Patentblatt 2015/47**

(62) Dokumentnummer(n) der früheren Anmeldung(en) nach Art. 76 EPÜ:
**13736506.0 / 2 709 718**

(73) Patentinhaber: **Baklayan, Alan, E.**
**80331 München (DE)**

(72) Erfinder: **Baklayan, Alan, E.**
**80331 München (DE)**

(74) Vertreter: **Flügel Preissner Schober Seidel Patentanwälte PartG mbB**
**Nymphenburger Strasse 20a**
**80335 München (DE)**

(56) Entgegenhaltungen:
**EP-A1- 1 529 550          US-A- 5 925 071**
**US-A1- 2005 090 867          US-A1- 2006 224 210**
**US-A1- 2007 156 178          US-A1- 2008 215 114**

**Beschreibung**

Die Erfindung betrifft eine Therapievorrichtung zur Erzeugung von elektromagnetischen Schwingungen.

Die Erfindung ist in Anspruch 1 definiert. Die abhängigen Ansprüche stellen bevorzugte Ausführungsformen dar.

Aus dem Stand der Technik sind Geräte zur Behandlung von Patienten mit energetischen Schwingungen bekannt. Beispielsweise DE 20 2010 010 700 U1 beschreibt ein solches Behandlungsgerät. Das Gerät erzeugt elektrische Schwingungen, die über ein Kabel an eine Elektrode geleitet werden. Der Patient berührt die Elektrode und die von dem Gerät erzeugten elektrischen Schwingungen werden in den Körper des Patienten geleitet. Die elektrischen Schwingungen wechselwirken mit elektrophysiologischen Schwingungen des Körpers. Elektrische Schwingungen treten in jedem Organismus auf. Die von dem Behandlungsgerät erzeugten elektrischen Schwingungen sollen die sich in dem Körper befindenden elektrophysiologischen Schwingungen anregen, verstärken oder unterdrücken, je nachdem, ob die elektrophysiologischen Schwingungen gewünscht oder nicht gewünscht sind. Nicht gewünschte elektrophysiologische Schwingungen können für Krankheiten oder andere Störungen des Organismus verantwortlich sein. Werden elektrophysiologische Schwingungen durch das Behandlungsgerät verstärkt, können positive Effekte der gewünschten elektrophysiologischen Schwingungen verstärkt werden.

US 2007/0156178 A1 offenbart eine Elektrotherapievorrichtung für den menschlichen Körper. Die Vorrichtung umfasst 2 Elektroden und eine Wechselspannungsquelle in Form eines Signalgenerators, wobei die Elektrode eine Erdungselektrode zu sein scheint. Die Elektroden werden mit Hilfe eines Triggersystems gesteuert um den Patienten am Herz zu behandeln. Dabei wird der Körper des Patienten in einem ersten Zeitraum mit einer Wechselspannung beaufschlagt und in einem zweiten Zeitraum wird die Stimulation unterbrochen.

EP 1 529 550 A1 offenbart eine Elektrotherapievorrichtung für den menschlichen Körper mit einer aktiven Elektrode und einer passiven Elektrode, wobei die passive Elektrode geerdet ist. Ein Mikroprozessor erzeugt mit Hilfe eines Transformators eine Wechselspannung zum behandeln des Herzens des Patienten. In einer Phase wird Wechselspannung beaufschlagt während in einer anderen Phase keine Stimulierung erfolgt. Um die Weite der negativen Halbflanke über seine gesamte Höhe konstant zu halten wird entsprechend einem Steuerungspuls die aktive Elektrode mit der passiven Elektrode kurzgeschlossen.

US 2005/0090867 offenbart eine Elektrotherapievorrichtung für den menschlichen Körper ähnlich der aus US 2007/0156178 A1. Die Vorrichtung umfasst 2 Elektroden und eine Wechselspannungsquelle in Form eines Signalgenerators, wobei die Elektrode eine Erdungselektrode zu sein scheint. Die Elektroden werden mit Hilfe eines Triggersystems gesteuert um den Patienten am Herz zu behandeln. Dabei wird der Körper des Patienten in einem ersten Zeitraum mit einer Wechselspannung beaufschlagt und in einem zweiten Zeitraum wird die Stimulation unterbrochen.

US 2008/0215114 A1 offenbart eine Elektrotherapievorrichtung zur Behandlung von Menschen oder Tieren. Die Vorrichtung umfasst zumindest eine aktive Elektrode und zumindest eine passive Elektrode. Ein Signalprozessor erzeugt eine Wechselspannung zur Behandlung des Patienten. Dabei wird der Körper des Patienten in einem ersten Zeitraum mit einer Wechselspannung beaufschlagt und in einem zweiten Zeitraum wird die Stimulation unterbrochen. Die Behandlung zielt dabei auf gesunde Menschen oder Tiere ab, die ihren mentalen oder physischen Zustand in bestimmten Aspekten verbessern wollen.

Der Erfindung liegt die Aufgabe zugrunde, eine Therapievorrichtung bereitzustellen, die eine besonders gute Behandlung eines Lebewesens ermöglicht.

Die Aufgabe wird durch die Therapievorrichtung nach Anspruch 1 gelöst. Vorteilhafte Ausgestaltungen sind Gegenstand der nebengeordneten Ansprüche.

Erfindungsgemäß umfasst die Therapievorrichtung zur Erzeugung von elektromagnetischen Schwingungen wenigstens eine Frequenzgeneratoreinheit. Die Frequenzgeneratoreinheit umfasst einen Frequenzgenerator zur Erzeugung von Wechselspannung, einen ersten Elektrodenausgang zum Anschluss einer ersten Elektrode und einen zweiten Elektrodenausgang zum Anschluss einer zweiten Elektrode. Der Frequenzgenerator weist einen Spannungsausgang auf, an dem die von dem Frequenzgenerator erzeugte Wechselspannung anliegt. Der Frequenzgenerator weist ferner einen Neutralausgang auf, der elektrisch neutral ist. Der erste Elektrodenausgang ist mit dem Spannungsausgang verbunden, während der zweite Elektrodenausgang mit dem Neutralausgang verbunden ist.

Die von der Therapievorrichtung erzeugten elektromagnetischen Schwingungen dienen vorzugsweise der Behandlung von Menschen oder Tieren mit den von dem Frequenzgenerator erzeugten elektrischen Schwingungen. Wie bereits oben dargestellt, lassen sich mit den elektromagnetischen Schwingungen gewünschte elektrophysiologische Effekte im Körper des Patienten verstärken oder abschwächen. Dazu berührt der Patient vorzugsweise eine erste Elektrode, die über ein Kabel mit dem ersten Elektrodenausgang verbunden ist. Der Patient kann die erste Elektrode halten oder die erste Elektrode kann an dem Patienten mittels eines Fixierbands oder Ähnlichem angebracht sein.

Vorzugsweise berührt der Patient auch eine zweite Elektrode, die über ein Kabel mit dem zweiten Elektrodenausgang verbunden ist. Die zweite Elektrode kann wie die erste Elektrode ausgestaltet sein. Die erste und/oder die zweite Elektrode können auch als eine Antenne zur Aussendung von elektromagnetischen Wellen ausgebildet sein.

Der Frequenzgenerator erzeugt eine Wechselspannung, die an dem Spannungsausgang anliegt. Über den ersten Elektrodenausgang wird die Wechselspannung an die erste Elektrode übertragen. Die Spannung kann vorzugsweise zwischen 0,1 V und 20 V, vorzugsweise zwischen 0,1 V und 16 V variiert werden. Der Neutralausgang des Frequenzgenerators ist vorzugsweise geerdet, kann aber auch mit einer konstanten Spannung beaufschlagt sein. Insbesondere kann der Neutralausgang mit einer Wechselspannung beaufschlagt werden, die gegenüber der am Spannungsausgang anliegenden Wechselspannung invertiert wird. Auf diese Weise kann die am Patienten anliegende Wechselspannung in ihrer Amplitude verdoppelt werden. Bevorzugt kann auch der Spannungsausgang mit einer Offsetspannung belegt sein, so dass die Wechselspannung beispielsweise nur mit einer positiven oder negativen Spannung oszilliert. Der Frequenzgenerator hat vorzugsweise einen Innenwiderstand von 100 Ω bis 100 kΩ, insbesondere von 1 kΩ.

Wenn der Patient sowohl die erste als auch die zweite Elektrode berührt, kann Strom zwischen den beiden Elektroden fließen. Es hat sich gezeigt, dass das Fließen von Strom für die elektrophysiologische Behandlung besonders wirkungsvoll ist. Durch entsprechendes Positionieren der Elektroden lässt sich der von dem Strom durchflossene Teil des Körpers des Patienten bestimmen, um die Wirkung der Behandlung in dem Körperteil je nach Bedarf zu erhöhen und zu vermindern.

Bevorzugt weist die Therapievorrichtung wenigstens einen Behälter zur Aufnahme einer Probe auf, der zwischen dem Spannungsausgang und dem ersten Elektrodenausgang geschaltet ist. Der Behälter ist vorzugsweise aus einem elektrisch leitenden Material, insbesondere Metall, hergestellt.

Der Behälter dient zur Aufnahme von Proben, die die von dem Frequenzgenerator erzeugte Wechselspannung auf eine für die Behandlung des Patienten positive Weise beeinflussen soll. Dies geschieht beispielsweise durch eine Veränderung der Kapazität des Behälters. Die geänderte Kapazität verändert dann die anliegende Wechselspannung. Die Proben können Eigenproben des Patienten, wie beispielsweise Blut oder Speichel, sein oder belastende Substanzen, wie beispielsweise Allergene, Schwermetalle, Viren oder Bakterien, enthalten.

Es ist bevorzugt, dass der erste Elektrodenausgang wenigstens zwei Anschlüsse, insbesondere drei Anschlüsse umfasst, wobei vorzugsweise der zweite Elektrodenausgang einen Anschluss umfasst, der mit einem der Anschlüsse des ersten Elektrodenausgangs eine koaxiale Steckverbindung, insbesondere einen BNC-Anschluss, bildet.

Es ist somit bevorzugt, dass die Therapievorrichtung zwei Anschlüsse, insbesondere Steckanschlüsse, aufweist, an denen die von dem Frequenzgenerator am Elektrodenausgang erzeugte Wechselspannung abgegriffen werden kann. Ferner hat die Therapievorrichtung vorzugsweise eine koaxiale Steckverbindung, bei der insbesondere der Mittelleiter mit dem Spannungsausgang des Frequenzgenerators verbunden ist und weiter vorzugsweise der Außenleiter mit dem Neutralausgang des Frequenzgenerators. An der koaxialen Steckverbindung können daher zwei Elektroden angeschlossen werden. Es ist auch möglich, dass an der koaxialen Steckverbindung lediglich mit dem Neutralausgang des Frequenzgenerators verbunden wird und die Spannung mittels der beiden anderen Anschlüsse des ersten Ausgangs verbunden ist. An der Therapievorrichtung ist vorzugsweise der Stecker der koaxialen Steckverbindung vorgesehen, wohingegen die Elektroden mit der Buchse der Steckverbindung verbunden sind. Eine umgekehrte Anordnung ist auch möglich. Die koaxiale Steckverbindung kann ein BNC-Anschluss, ein SMA-Anschluss oder eine andere Steckverbindung sein, die insbesondere für Hochfrequenzanschlüsse geeignet sind.

Es ist bevorzugt, dass die Elemente der Frequenzgeneratoreinheit innerhalb eines Gehäuses eingebaut sind, wobei die Anschlüsse von außen zugänglich sind. Insbesondere ist auch der Behälter im Gehäuse angebracht, so dass die Proben in den Behälter eingebracht werden können, ohne das Gehäuse zu öffnen. Der Behälter kann vorzugsweise mittels eines Deckels verschlossen werden.

Bevorzugt weist die Therapievorrichtung einen Energiespeicher, insbesondere einen Akkumulator auf, der den Frequenzgenerator mit elektrischer Energie versorgt.

Es ist somit bevorzugt möglich, dass die Therapievorrichtung von einem Stromnetz getrennt betrieben werden kann. Zum einen ermöglicht dies, dass die durch das Stromnetz erzeugte elektromagnetische Schwingung, wie zum Beispiel die 50Hz-Strahlung, vermieden werden kann, und zum anderen benötigt die Therapievorrichtung vorzugsweise keine Sicherungsmaßnahmen, die bei medizinischen Geräten, die an das Stromnetz angeschlossen werden, notwendig sind. Insbesondere die Vermeidung von elektromagnetischen Schwingungen in der Nähe des Patienten hat den positiven Effekt, dass keine externen Schwingungen die Behandlung des Patienten beeinflussen.

Der Energiespeicher kann insbesondere eine wiederaufladbare Batterie sein, die mittels eines Ladegeräts, das an die Therapievorrichtung anschließbar ist, aufgeladen werden kann. Ferner hat das Vorsehen eines Energiespeichers den Vorteil, dass die Therapievorrichtung variabler einsetzbar ist, da sie nicht an das Vorhandensein eines elektrischen Stromnetzes geknüpft ist. Der Energiespeicher ist vorzugsweise auch innerhalb des Gehäuses der Therapievorrichtung angeordnet.

Es ist bevorzugt, dass der Frequenzgenerator zur Erzeugung von Wechselspannung mit unterschiedlichen Frequenzen, insbesondere zwischen 0,1 Hz und 100 MHz, ausgebildet ist, wobei vorzugsweise der Frequenzgenerator geeignet ist, die Spannung und die Art der ausgegebenen Wechselspannung zu verändern.

Die Abdeckung eines großen Frequenzbereichs zwischen 0,1 Hz und 100 MHz, insbesondere zwischen 1 Hz und 10 MHz, hat den Vorteil, dass die Therapievorrichtung in verschiedensten Anwendungsbereichen verwendet werden kann. Die Spannung, die Spitzen-Spitzen-Spannung, die die Amplitudenspannung oder die Effektivspannung, kann vorzugsweise zwischen 0,1 V und 16 V variiert werden. Vorzugsweise kann der Frequenzgenerator eine Wechselspannung mit sinuswellenförmigem, rechteckigem oder dreieckigem Wellenprofil erzeugen. Die einzelnen Wellenprofile haben erfahrungsgemäß jeweils besondere Vorteile bei den jeweiligen Behandlungen. Ferner kann eine monofrequente, sinusförmige Wechselspannung durch Addition einer oder mehrerer Oberschwingungen (bis zur Entstehung einer Rechtecksspannung) mit Hilfe des Frequenzgenerators verändert werden.

Es ist bevorzugt, dass die Therapievorrichtung eine Steuereinrichtung aufweist, welche mit dem Frequenzgenerator verbunden ist, wobei die Frequenz, die Spannung und/oder die Art der ausgegebenen Wechselspannung mittels der Steuereinrichtung steuerbar sind.

Vorzugsweise ist die Steuereinrichtung innerhalb des Gehäuses der Therapievorrichtung angeordnet und insbesondere durch einen Mikroprozessor realisiert. Weiter vorzugsweise ist der Energiespeicher mit der Steuereinrichtung verbunden, so dass der Frequenzgenerator über die Steuereinrichtung mit Energie versorgt wird. Die Steuereinrichtung gibt vorzugsweise die Frequenz, die Spannung und/oder die Art der ausgegebenen Wechselspannung an den Frequenzgenerator aus, der wiederum eine Wechselspannung mit den von der Steuereinrichtung angegebenen Parametern ausgibt.

Es ist bevorzugt, dass die Therapievorrichtung eine Anzeigeeinrichtung und/oder eine Eingabeeinrichtung aufweist. Die Anzeigeeinrichtung zeigt die Frequenz, die Spannung und/oder die Art der ausgegebenen Wechselspannung an, wobei vorzugsweise die Frequenz, die Spannung und/oder die Art der ausgegebenen Wechselspannung mittels der Eingabeeinrichtung einstellbar sind.

Die Eingabeeinrichtung ist vorzugsweise mit der Steuereinrichtung verbunden. Ferner kann die Steuereinrichtung mit der Anzeigeeinrichtung, die beispielsweise ein Display sein kann, verbunden sein und diese ansteuern. Die Anzeigeeinrichtung und die Eingabeeinrichtung sind vorzugsweise an dem Gehäuse der Therapievorrichtung vorgesehen. Die Art der Wechselspannung kann beispielsweise durch einen Begriff oder durch eine visuelle Darstellung der Wechselspannung angezeigt werden. Die Frequenz und/oder die Spannung kann mittels einer numerischen Eingabe eingegeben werden. Es ist aber auch möglich, dass die Steuereinrichtung einen Speicher aufweist, in dem verschiedenste Frequenzen eingespeichert sind, die jeweils mit einer Nummer versehen sind. Durch Eingabe einer Nummer an der Eingabeeinrichtung kann die gewünschte Frequenz eingegeben werden.

Alternativ oder weiter bevorzugt ist der Frequenzgenerator zur Erzeugung von wenigstens zwölf festgelegten Frequenzen ausgebildet. Die Frequenzen liegen in einem Frequenzbereich, der eine untere Grenzfrequenz und eine obere Grenzfrequenz aufweist. Die obere Grenzfrequenz ist doppelt so groß wie die untere Grenzfrequenz, wobei vorzugsweise die obere Grenzfrequenz nicht Teil des Frequenzbereichs ist.

Bevorzugt ist der Frequenzgenerator ausgebildet, zumindest zwölf festgelegte, unterschiedliche erste Frequenzen zu erzeugen. Diese zwölf Frequenzen liegen alle in innerhalb eines ersten Bereichs, dessen Grenzen bevorzugt einem Oktavengesetz folgen. So ist die untere Grenzfrequenz gleich der niedrigsten Frequenz der zwölf ersten Frequenzen. Die obere Grenzfrequenz ist durch die doppelte Frequenz der unteren Grenzfrequenz gebildet, so dass sich ein oktavenspezifisches Verhältnis von 2:1 ergibt. Erfindungsgemäß ist die obere Grenzfrequenz aus dem ersten Bereich ausgeschlossen.

Es ist bevorzugt, dass das Verhältnis der Frequenzen zur unteren Grenzfrequenz 1, 14:13, 9:8, 6:5, 5:4, 4:3, 7:5, 3:2, 8:5, 5:3, 9:5 oder 13:7 beträgt. Ein Verhältnis der Frequenz zur unteren Grenzfrequenz von 2:1 entspricht der ersten Oberschwingung der unteren Grenzfrequenz und kann auch als Frequenz ausgewählt werden. Diese Frequenzverhältnisse entsprechen der Harmonikalischen Lehre und sind besonders geeignet, die Meridiane des Körpers anzuregen. Alternativ kann ein weiteres Frequenzverhältnis gewählt werden. Bei diesem unterscheiden sich benachbarte Frequenzen der zwölf Frequenzen, also die erste zu der zweiten, die zweite zu der dritten und in diesem Sinne weiter, um ein konstantes Verhältnis $\sqrt[12]{2}$ . Auch hier ergibt sich ein Verhältnis von Grundfrequenz zu erster Oberschwingung von 2:1.

Gemäß einem weiteren Aspekt der Erfindung umfasst eine Therapievorrichtung zur Erzeugung von elektromagnetischen Schwingungen wenigstens eine Frequenzgeneratoreinheit. Die Frequenzgeneratoreinheit umfasst einen Frequenzgenerator zur Erzeugung von Wechselspannung, einen ersten Elektrodenausgang zum Anschluss einer ersten Elektrode und einen zweiten Elektrodenausgang zum Anschluss einer zweiten Elektrode sowie eine Steuereinrichtung. Die Steuereinrichtung ist eingerichtet, den Frequenzgenerator für einen ersten Zeitraum zu aktivieren und für einen zweiten Zeitraum zu deaktivieren, wobei vorzugweise sich der erste Zeitraum und der zweite Zeitraum periodisch abwechseln. Die Therapievorrichtung weist vorzugsweise die oben genannten Merkmale und/oder Vorteile auf.

Durch das Aktivieren und Deaktivieren des Frequenzgenerators kann der Patient in dem ersten Zeitraum behandelt werden, während in dem zweiten Zeitraum der Patient zur Ruhe kommen kann. Besonders vorteilhaft ist, dass der Patient während des zweiten Zeitraums entladen werden kann, wenn er eine an mit dem Neutralausgang verbundene Handelektrode berührt. Die während des ersten Zeitraums aufgebrachte Ladung und durch Umwelteinflüsse aufgebrachte Ladung können in diesem Fall im zweiten Zeitraum vollständig abfließen. Vorzugsweise ist die Dauer des ersten und/oder des zweiten Zeitraums einstellbar.

Eine derartige Therapievorrichtung stellt die Grundlage für eine effektive Behandlung eines Patienten dar. Beispielsweise können die zwölf Frequenzen den zwölf Hauptmeridianen der Traditionellen Chinesischen Medizin zugeordnet werden, wodurch es dem behandelnden Therapeuten vereinfacht wird, diese Meridiane mit entsprechenden Frequenzen zu aktivieren.

Bei einer bevorzugten Ausführungsform der Erfindung ist die Frequenzgeneratoreinheit ausgebildet, die ersten Frequenzen in konstanten Verhältnissen zueinander zu erzeugen. Die Verhältnisse müssen dabei nicht zwangsläufig identisch sein, sind jedoch bevorzugt konstant, so dass bei Variation einer der zwölf ersten Frequenzen sämtliche weiteren Frequenzen der ersten Frequenzen gemäß den bevorzugten Verhältnissen ebenso geändert werden.

Weiterhin sind die Verhältnisse der ersten Frequenzen untereinander derart ausgebildet, dass sie gleich zu den Verhältnissen sind, die die zwölf Halbtöne einer Oktave der Klanglehre aufweisen. Durch diese Gesetzmäßigkeiten ist es möglich, eine "Tonleiter" der ersten Frequenzen aufzustellen, die der Tonleiter einer Oktave mit 12 Halbtönen gleicht.

Weiterhin ist bevorzugt vorgesehen, dass die ersten Frequenzen zueinander ein Verhältnis aufweisen, das identisch mit einem Halbton und/oder einem Viertelton aus der Klanglehre ist. Die Therapievorrichtung kann daher diskrete Frequenzwerte abgeben, so dass dem Therapeuten die Auswahl der für den aktuellen Patienten passenden Frequenz erleichtert wird.

Bei einer weiteren vorteilhaften Ausführungsform der Erfindung ist die Frequenzgeneratoreinheit ausgebildet, neben den zwölf ersten Frequenzen weitere Frequenzen zu erzeugen. Bevorzugt folgen die weiteren Frequenzen denselben Gesetzmäßigkeiten wie die ersten Frequenzen. Somit liegen die weiteren Frequenzen innerhalb eines weiteren Bereichs, der durch die niedrigste der weiteren Frequenzen nach unten begrenzt wird. Weiterhin ist auch für jeden weiteren Bereich die Obergrenze als doppelte Frequenz der Frequenz der Untergrenze definiert. Auf diese Weise sind die Voraussetzungen vorhanden, die weiteren Frequenzen analog zu aus der Klanglehre bekannten Oktaven um den ersten Bereich herum anzuordnen. Jede weitere Frequenz steht somit in einem festen Verhältnis zu einer der ersten Frequenzen, wobei die jeweils größere Frequenz um ein natürliches Vielfaches von 2 größer als die jeweils kleinere Frequenz ist. Dadurch ergibt sich ein aus der Klanglehre bekanntes Bild, in dem der gesamte Frequenzraum in eine Aneinanderreihung von oktavenähnlichen Bereichen aufgeteilt ist.

Eine derartige Oktavierung der möglichen Frequenzen, mit denen ein Patient behandelt werden kann, macht es für den Therapeuten einfacher, eine zu verwendende Frequenz für die Behandlung zu finden. So kann beispielsweise eine Frequenz, die einem bestimmten Meridian zugeordnet ist, als Ton angesehen werden, der jedoch auch in anderen Oktaven vorkommt. Dies bedeutet, dass beispielsweise eine doppelt oder halb so große Frequenz auf denselben Meridian wirkt. Dem Therapeuten steht somit offen, eine bestmöglichste Frequenz gemäß dem Oktavengesetz auszuwählen.

Bevorzugt ist die obere Grenzfrequenz und/oder die untere Grenzfrequenz des ersten Bereichs bevorzugt manuell anpassbar. Alternativ oder zusätzlich ist die obere Grenzfrequenz und/oder die untere Grenzfrequenz des ersten Bereichs bevorzugt mittels einer automatischen Steuereinrichtung einstellbar.

Die Anpassbarkeit der Grenzen erlaubt eine vorteilhafte Kalibrierung der Therapievorrichtung auf einen bestimmten Patienten. Da die ersten Frequenzen bei allen Patienten meist nicht exakt gleich sind, kann hier der Therapeut korrigierend eingreifen und die ersten Frequenzen und damit den ersten Bereich entsprechend anpassen. Da die Verhältnisse der Frequenzen untereinander bevorzugt konstant sind, verschiebt sich somit lediglich die Frequenzlage als Ganzes, während die grundsätzliche Oktavierung erhalten bleibt.

Es ist bevorzugt, dass die Therapievorrichtung eine zweite Frequenzgeneratoreinheit und insbesondere eine dritte Frequenzgeneratoreinheit aufweist, wobei vorzugsweise die zweite Frequenzgeneratoreinheit und die dritte Frequenzgeneratoreinheit gegeneinander und/oder gegenüber der ersten Frequenzgeneratoreinheit galvanisch getrennt sind.

Die zweite und/oder die dritte Frequenzgeneratoreinheit sind vorzugsweise innerhalb des Gehäuses der Therapievorrichtung angeordnet. Die zweite und/oder die dritte Frequenzgeneratoreinheit sind vorzugsweise wie die oben beschriebene Frequenzgeneratoreinheit ausgebildet und weisen demnach insbesondere jeweils einen Frequenzgenerator, einen ersten Elektrodenausgang und einen zweiten Elektrodenausgang auf, wobei der Frequenzgenerator jeweils einen Spannungsausgang und einen Neutralausgang aufweist. Ferner sind vorzugsweise jeder Frequenzgeneratoreinheit die oben genannten Anschlüsse zugeordnet. Die einzelnen Frequenzgeneratoreinheiten sind voneinander galvanisch (elektrisch) getrennt, so dass sich die Frequenzgeneratoren der jeweiligen Frequenzgeneratoreinheiten nicht gegenseitig beeinflussen können. Eine gegenseitige elektrische Störung, die ungewünschte Interferenzen zur Folge haben kann, kann somit vermieden werden. Vorzugsweise sind die einzelnen Frequenzgeneratoreinheiten zusätzlich gegen elektromagnetische Strahlung abgeschirmt.

Die zweite und/oder dritte Frequenzgeneratoreinheit wird bevorzugt durch die Steuereinrichtung gesteuert. Auch die Energieversorgung sowie die Eingabe und Anzeige der von den Frequenzgeneratoren der zweiten und dritten Frequenzgeneratoreinheit folgen ähnlichen Mechanismen, wie sie im Zusammenhang mit der ersten Frequenzgeneratoreinheit beschrieben worden sind.

Es ist ferner bevorzugt, dass die Steuereinrichtung geeignet ist, die Frequenz, die Spannung und/oder die Art der ausgegebenen Wechselspannung der Frequenzgeneratoren der zweiten Frequenzgeneratoreinheit und/oder der dritten Frequenzgeneratoreinheit zu steuern.

Vorzugsweise hat die von der zweiten Frequenzgeneratoreinheit und/oder dritten Frequenzgeneratoreinheit erzeugte Wechselspannung eine Frequenz, die das $2^Z$-fache der Frequenz der von der ersten Frequenzgeneratoreinheit erzeugten Wechselspannung ist. z ist dabei eine ganze (positive oder negative) Zahl. Das heißt, dass die von der zweiten Frequenzgeneratoreinheit erzeugte Frequenz das Doppelte, Vierfache usw. oder das Einhalbfache, Einviertelfache usw. der Frequenz der von der ersten Frequenzgeneratoreinheit erzeugten Wechselspannung ist. Die Frequenz der von der dritten Frequenzgeneratoreinheit erzeugten Wechselspannung unterscheidet sich von der Frequenz der von der ersten Frequenzgeneratoreinheit erzeugten Wechselspannung auf gleiche Weise. Insbesondere kann die Steuereinrichtung so eingestellt werden, dass bei Änderung der Frequenz der von der ersten Frequenzgeneratoreinheit erzeugten Wechselspannung auch die Frequenzen der von der zweiten oder dritten Frequenzgeneratoreinheit erzeugten Wechselspannung geändert werden. Vorzugsweise ist die Art der Wechselspannung dabei bei allen Frequenzgeneratoreinheiten gleich.

Es ist bevorzugt, dass die Phase der von dem Frequenzgenerator der zweiten Frequenzgeneratoreinheit und/oder dem Frequenzgenerator der dritten Frequenzgeneratoreinheit ausgegebenen Wechselspannung zur Phase der von dem Frequenzgenerator der ersten Frequenzgeneratoreinheit ausgegebenen Wechselspannung verschoben ist, insbesondere um 60°, 90°, 120° oder 180°.

Wenn ein Patient zwei und/oder drei Elektroden, die jeweils mit den verschiedenen Frequenzgeneratoreinheiten verbunden sind, berührt, hat sich gezeigt, dass die Invertierung der Wechselspannungen der einzelnen Frequenzgeneratoreinheiten einen besonders starken therapeutischen Effekt auf den Patienten hat. Beispielsweise gibt die Frequenzgeneratoreinheit eine Wechselspannung mit einer bestimmten Frequenz aus, die zu der von der zweiten Frequenzgeneratoreinheit erzeugten Wechselspannung mit der gleichen Frequenz um 180° phasenverschoben ist. Die von der dritten Frequenzgeneratoreinheit erzeugte Wechselspannung kann gegenüber der von der ersten oder der zweiten Frequenzgeneratoreinheit erzeugten Wechselspannung um 60°, 90°, 120° oder 180° phasenverschoben sein.

Es ist bevorzugt, dass sich die Spannung der von dem Frequenzgenerator der zweiten Frequenzgeneratoreinheit ausgegebenen Wechselspannung und/oder der von dem Frequenzgenerator der dritten Frequenzgeneratoreinheit ausgegebenen Wechselspannung von der Spannung der von dem Frequenzgenerator der ersten Frequenzgeneratoreinheit ausgegebenen Wechselspannung unterscheidet.

Vorzugsweise haben die Spannungen der drei ausgegebenen Frequenzen ein Verhältnis von 4 : 2 : 1. Es sind aber auch andere Frequenzverhältnisse zwischen den verschiedenen Wechselspannungen möglich. Insbesondere können die Spannungen der von den drei Frequenzgeneratoreinheiten erzeugten Wechselspannungen auch gleich sein. Vorzugsweise lassen sich die Spannungen und damit auch die Verhältnisse der Spannungen der von den drei Frequenzgeneratoreinheiten erzeugten Wechselspannungen einstellen, um einen optimalen Behandlungserfolg erzielen zu können.

Es ist bevorzugt, dass die Steuereinrichtung einen Regler umfasst, welcher die Spannung der Wechselspannung derart regelt, dass der Stromfluss zwischen dem ersten Elektrodenausgang und dem zweiten Elektrodenausgang zumindest im Mittel konstant ist, wobei vorzugsweise die Stromstärke einstellbar ist.

Es hat sich gezeigt, dass die Höhe des am oder im Patienten fließenden Stroms wichtig für den Behandlungserfolg ist. Allerdings unterscheidet sich die Leitfähigkeit der Elektrode zu der Haut des Patienten von Patient zu Patient und von Situation zu Situation, da die Beschaffenheit der Haut bei jedem Patienten unterschiedlich ist und die Leitfähigkeit maßgeblich von der Feuchtigkeit zwischen der Haut und der Elektrode abhängt. Um diese Faktoren nicht berücksichtigen zu müssen, kann vorzugsweise die Stromstärke, insbesondere mit Hilfe der Eingabeeinrichtung, an der Therapievorrichtung eingestellt werden. Der Regler übernimmt dann das Festlegen der Spannung. Bei einer guten Leitfähigkeit zwischen der Elektrode und der Haut des Patienten, das heißt bei einem geringern Kontaktwiderstand, wird die am Frequenzgenerator am Spannungsausgang anliegende Spannung geringer sein, als wenn der Kontaktwiderstand zwischen Elektrode und Haut groß ist.

Ferner stellt die Erfindung ein Verfahren zur Steuerung einer Therapievorrichtung bereit, die einen ersten Frequenzgenerator und einen zweiten Frequenzgenerator umfasst. Die Schritte des Verfahrens lauten Steuern des ersten Frequenzgenerators zur Erzeugung einer ersten Wechselspannung mit einer ersten Frequenz, einer ersten Spannung und einer ersten Phase, Steuern des zweiten Frequenzgenerators zur Erzeugung einer zweiten Wechselspannung mit einer zweiten Frequenz, einer zweiten Spannung und einer zweiten Phase, und Einstellen der ersten Phase im Vergleich zu der zweiten Phase, dass sich die Phasen um 160° bis 200°, insbesondere um 180°, unterscheiden.

Vorzugsweise lassen sich damit die oben beschriebene Invertierung und die damit verbundenen Erfolge erreichen. Das Verfahren ist bevorzugt dazu geeignet, die Therapievorrichtung, wie sie oben oder in Anspruch 1 definiert ist, zu steuern. Dann entspricht der erste und zweite Frequenzgenerator dem ersten und zweiten Frequenzgenerator der ersten und zweiten Frequenzgeneratoreinheit. Ferner kann das Verfahren auch dazu verwendet werden, drei Frequenzgeneratoren zu steuern. In einem solchen Fall unterscheidet sich dann die Phase der von einem dritten Frequenzgenerator (einer dritten Frequenzgeneratoreinheit) erzeugte Wechselspannung zu der ersten und zweiten Wechselspannung um 0°, 30°, 60°, 90°, 120°, 150°, 180° und/oder dazwischen liegende Werte.

Es ist bevorzugt, dass sich das Verfahren durch Einstellen der ersten Frequenz und der zweiten Frequenz, so dass das Verhältnis der ersten Frequenz zu der zweiten Frequenz gleich $2^Z$ ist, wobei z eine ganze Zahl ist, auszeichnet.

Dieses Verhältnis, so wie es bereits oben beschrieben wurde, hat sich in der Praxis durch die Erzielung besonders guter Behandlungserfolge bewährt.

Bevorzugt hat das Verfahren den Schritt Einstellen einer Frequenz des ersten Frequenzgenerators aus einer von wenigstens zwölf festgelegten Frequenzen, wobei die Frequenzen in einem Frequenzbereich liegen, der eine untere Grenzfrequenz und eine obere Grenzfrequenz aufweist, wobei die obere Grenzfrequenz doppelt so groß ist wie die untere Grenzfrequenz, und wobei vorzugsweise die obere Grenzfrequenz nicht Teil des Frequenzbereichs ist.

Bevorzugt hat das Verfahren den Schritt Einstellen der ersten Frequenz mit einem Verhältnis von 1, 14:13, 9:8, 6:5, 5:4, 4:3, 7:5, 3:2, 8:5, 5:3, 9:5 oder 13:7 zu der unteren Grenzfrequenz. Vorzugsweise gelten für diese Schritt auch die hinsichtlich der Therapievorrichtung angestellten Überlegengen und die genannten Merkmale.

Ferner stellt die Erfindung ein Verfahren zur Steuerung einer Therapievorrichtung bereit, die einen ersten Frequenzgenerator und einen zweiten Frequenzgenerator umfasst. Die Schritte des Verfahrens lauten Steuern des ersten Frequenzgenerators zur Erzeugung einer ersten Wechselspannung mit einer ersten Frequenz, einer ersten Spannung und einer ersten Phase, Steuern des zweiten Frequenzgenerators zur Erzeugung einer zweiten Wechselspannung mit einer zweiten Frequenz, einer zweiten Spannung und einer zweiten Phase, und Aktivieren des Frequenzgenerators für einen ersten Zeitraum und Deaktivieren des Frequenzgenerators für einen zweiten Zeitraum, wobei vorzugweise sich der erste Zeitraum und der zweite Zeitraum periodisch abwechseln.

Bevorzugt weist das Verfahren die oben beschriebenen Merkmale und/oder Vorteile auf. Ferner gelten vorzugsweise die hinsichtlich der Therapievorrichtung angestellten Überlegungen und Merkmale.

Die zwölf Frequenzen entsprechen in Ihrem Frequenzverhältnis bevorzugt dem Frequenzverhältnis der Töne einer Tonleiter, wie dies zuvor beschrieben wurde. Vorzugsweise hat wird die Frequenz des zweiten Frequenzgenerators als das $2^Z$-fache der Frequenz des ersten Frequenzgenerators eingestellt.

Im Folgenden wird die Erfindung anhand eines Ausführungsbeispiels in Verbindung mit den Zeichnungen beschrieben. Dabei zeigt:

Fig. 1    eine schematische Darstellung einer Therapievorrichtung;

Fig. 2    eine schematische Darstellung der Therapievorrichtung bei Behandlung eines Patienten

Fig.3    eine perspektivische Ansicht der Therapievorrichtung;

Fig. 4    eine weitere perspektivische Ansicht der Therapievorrichtung;

Fig. 5    eine Ansicht einer Anzeigeeinrichtung der Therapievorrichtung; und

Fig. 6a, b und c    eine Tabelle der in einer Steuereinrichtung der Therapievorrichtung gespeicherten Frequenzen.

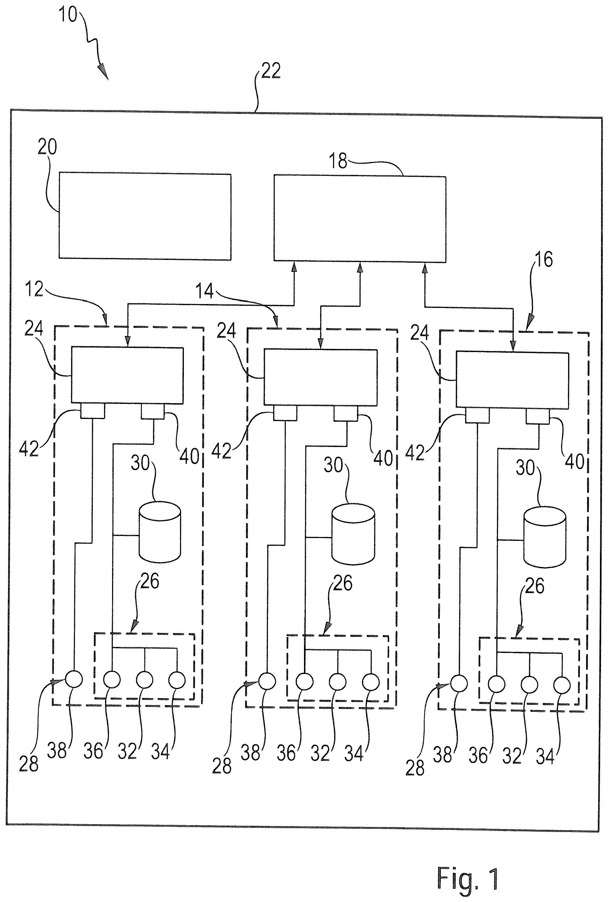

Bezug nehmend auf Fig. 1 hat eine Therapievorrichtung 10 eine erste Frequenzgeneratoreinheit 12, eine zweite Frequenzgeneratoreinheit 14, eine dritte Frequenzgeneratoreinheit 16, eine Steuereinrichtung 18 und einen Energiespeicher 20. Die genannten Elemente der Therapievorrichtung 10 sind in einem Gehäuse 22 angeordnet. Die drei Frequenzgeneratoreinheiten 12, 14 und 16 sind gegeneinander elektrisch isoliert und jeweils nur mit der Steuereinrichtung 18 verbunden.

Stellvertretend für die drei Frequenzgeneratoreinheiten soll die erste Frequenzgeneratoreinheit beschrieben werden. Die erste Frequenzgeneratoreinheit 12 hat einen Frequenzgenerator 24, einen ersten Elektrodenausgang 26, einen zweiten Elektrodenausgang 28 sowie einen Behälter 30. Der erste Elektrodenausgang 26 hat einen Handelektrodenanschluss 32, einen Abgreifelektrodenanschluss 34 und einen Leiteranschluss 36. Der zweite Elektrodenausgang hat einen Neutralanschluss 38. An die Anschlüsse 32, 34, 36 und 38 kann jeweils eine Elektrode 39 angeschlossen werden. Der Leiter-Anschluss 36 und der Neutralanschluss 38 bilden einen BNC-Anschluss, wobei der Neutralanschluss 38 der Außenleiter des BNC-Anschlusses ist.

Der Frequenzgenerator 24 hat einen Spannungsausgang 40, an dem die von dem Frequenzgenerator 24 erzeugte Wechselspannung anliegt, und einen Neutralausgang 42, der elektrisch neutral ist. Der Spannungsausgang 40 ist mit einem Behälter 30 und dem ersten Elektrodenausgang 26 verbunden. Demnach ist der Spannungsausgang 40 mit dem Handelektrodenanschluss 32, dem Abgreifelektrodenanschluss 34 und dem BNC-Anschluss 36 verbunden. Der Neutralausgang 42 ist mit dem zweiten Elektrodenausgang 28 und damit auch mit dem Neutralanschluss 38 verbunden.

Der Behälter 30 ist aus Metall hergestellt und kann mittels eines Deckels 43 verschlossen werden. Der Behälter 30 ist in dem Gehäuse 22 angeordnet. Nach Abnahme des Deckels 43 kann eine Probe in den Behälter 30 gestellt werden. An den Handelektrodenanschluss 32 kann beispielsweise eine Elektrode 39 angeschlossen werden, die von dem Patienten in der Hand gehalten werden kann. Allerdings sind auch andere Elektroden denkbar.

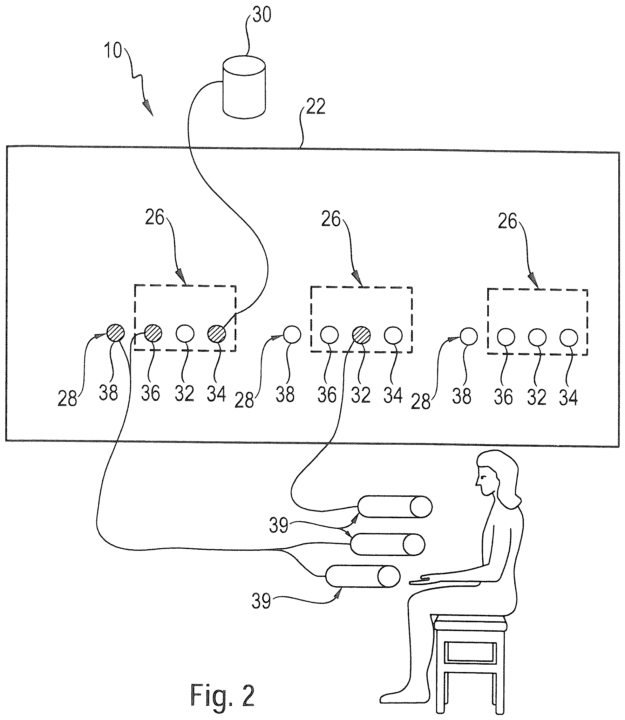

Wie in Fig. 2 zu erkennen ist, berührt ein Patient drei Elektroden 39, die als metallisches Rohr ausgeführt sind. Zwei der Elektroden 39 sind über ein BNC-Kabel mit dem BNC-Anschluss verbunden, das heißt, die beiden Elektroden sind mit dem Leiteranschluss 36 und dem Neutralanschluss verbunden. Die dritte gezeigte Elektrode 39 ist mit dem Handelektrodenanschluss 32 einer zweiten Frequenzgeneratoreinheit 12 verbunden. Die an den Elektroden anliegende Spannung kann sich in ihrer Art, Amplitude und Phase unterscheiden.

An dem Abgreifelektrodenanschluss 34 kann beispielsweise ein weiterer Behälter 30 zur Aufnahme von Proben angeschlossen werden, wie dies in Fig. 2 ersichtlich ist.

Der Energiespeicher 20, der in dieser Ausführungsform ein wiederaufladbarer Akkumulator ist, ist mit der Steuereinrichtung 18 verbunden und versorgt diese mit elektrischer Energie. Die Steuereinrichtung 18 ist wiederum mit jeder der Frequenzgeneratoreinheiten 12, 14 und 16 verbunden und steuert jeweils die erste Frequenzgeneratoreinheit 12, die zweite Frequenzgeneratoreinheit 14 und die dritte Frequenzgeneratoreinheit 16. Ferner werden die Frequenzgeneratoreinheiten 12, 14 und 16 über die Steuereinrichtung 18 mit elektrischer Energie versorgt.

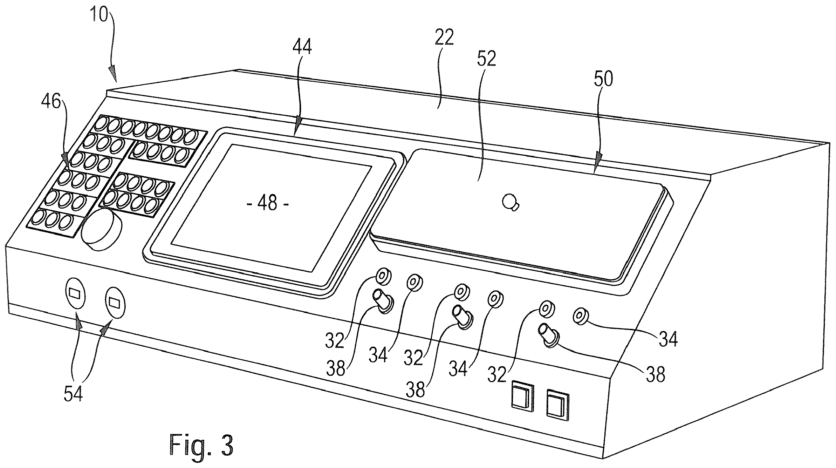

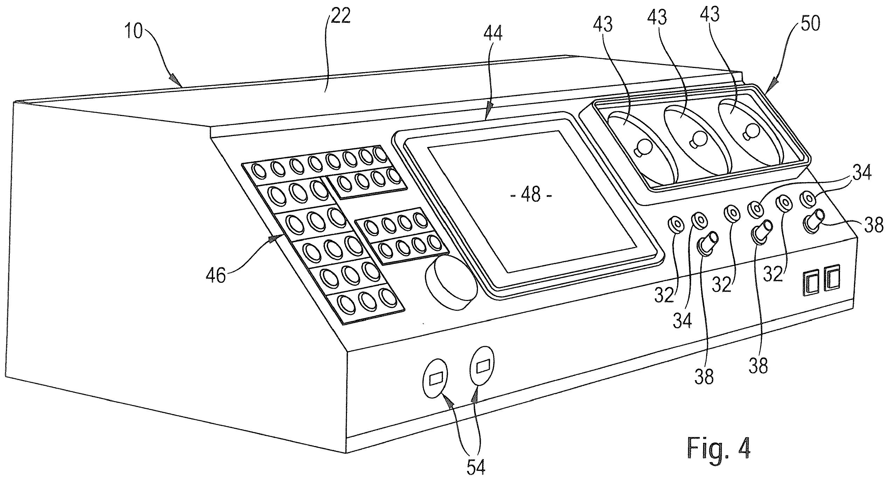

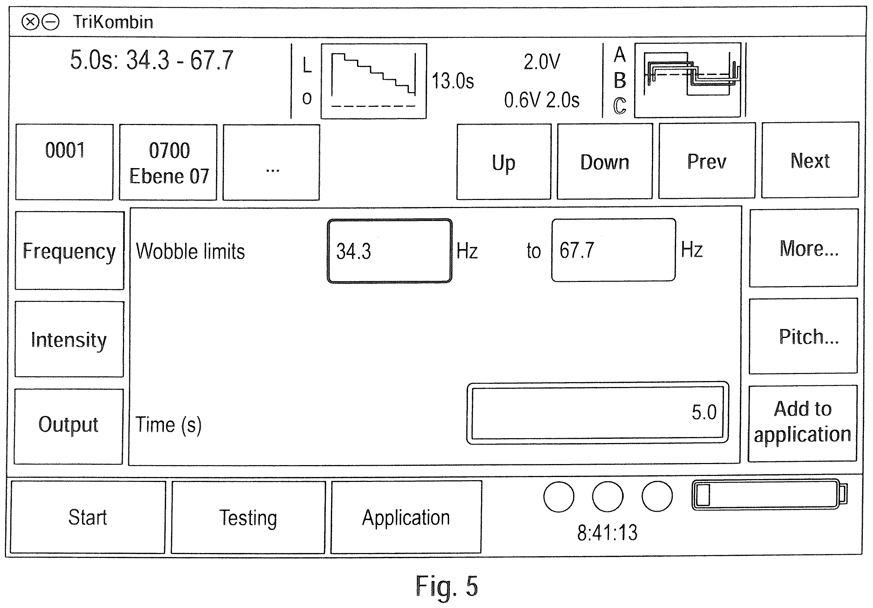

Wie aus Fig. 3 und 4 sichtbar ist, hat die Therapievorrichtung 10 ferner eine Anzeigeeinrichtung 44 und eine Eingabeeinrichtung 46. Die Anzeigeeinrichtung 44 umfasst ein Display 48, das in Fig. 5 vergrößert dargestellt ist. Auf dem Display 48 werden die Frequenz, die Spannung und die Art der Wechselspannung angegeben, die von den drei Frequenzgeneratoreinheiten 12, 14, und 16 ausgegeben wird. Ferner wird auch die Phasenbeziehung der Wechselspannung der von den drei Frequenzgeneratoreinheiten 12, 14 und 16 ausgegebenen Wechselspannungen graphisch dargestellt. Der Steuerungscomputer kann auch eine Wobbelung der Frequenz steuern, die auch auf dem Display 48 angezeigt wird. Ferner wird auch der Ladezustand des Energiespeichers 20 auf dem Display angezeigt. Die Einstellung erfolgt über Knöpfe der Eingabeeinrichtung 46. Die Tasten der Eingabeeinrichtung können sowohl eine Eingabe ermöglichen als auch durch das auf dem Display 48 angezeigte Menü führen.

Die Therapievorrichtung 10 hat auch einen Behälterzugang 50, in welchem die Behälter 30 der drei Frequenzgeneratoreinheiten 12, 14 und 16 angeordnet sind. Der Behälterzugang 50 kann durch eine Klappe 52 geschlossen werden. Die Therapievorrichtung hat außerdem einen Ladegerätanschluss 54, an welchem ein nicht gezeigtes Ladegerät angeschlossen werden kann, mittels welchem der Energiespeicher 20 geladen werden kann.

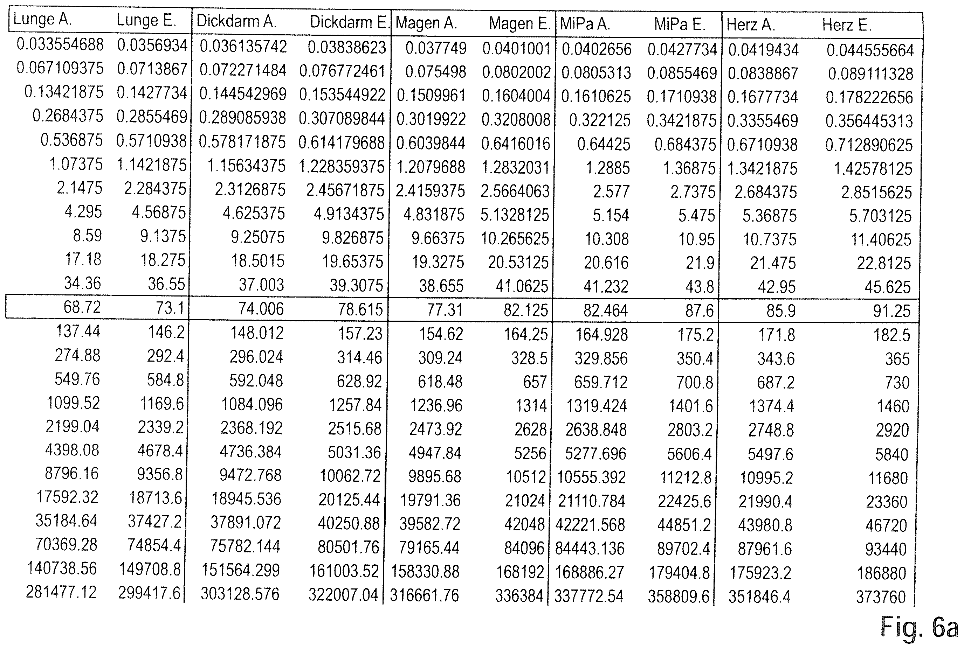

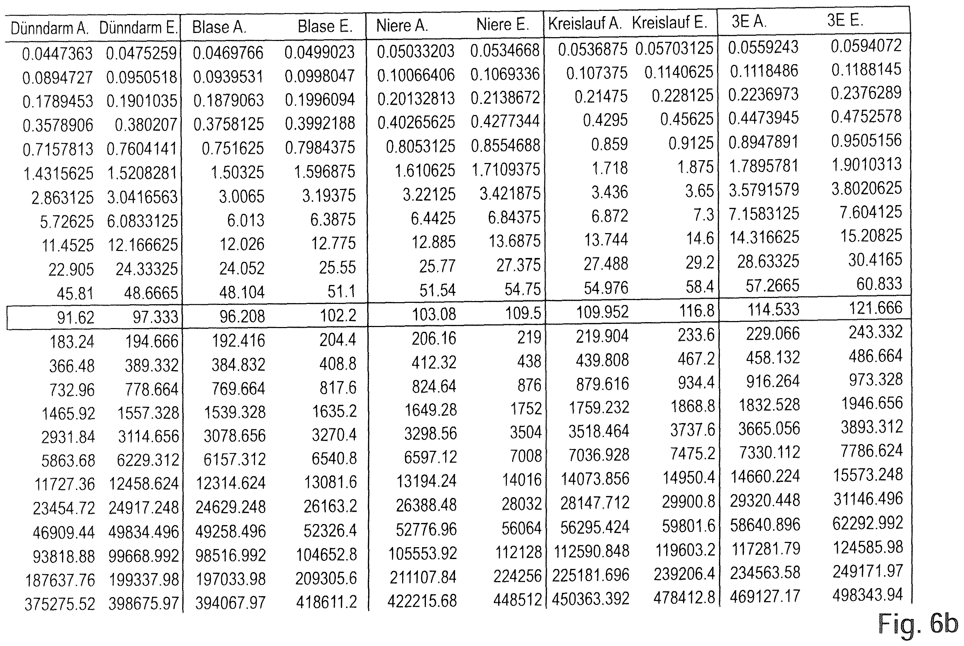

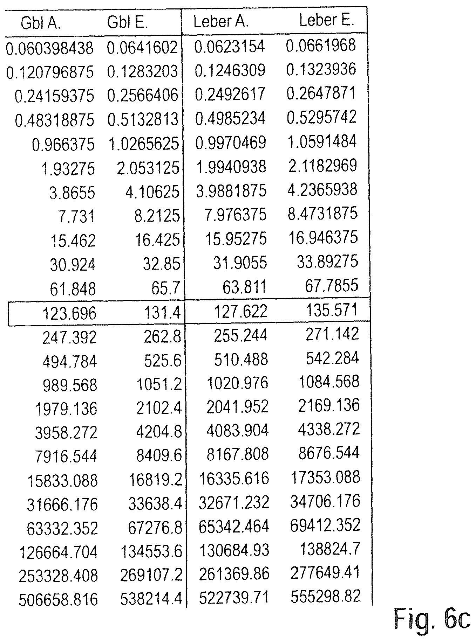

Fig. 6 zeigt eine Tabelle mit Frequenzen, die sich als besonders geeignet für die Behandlung mit elektromagnetischen Schwingungen erwiesen haben. Jede Spalte ist einem Meridian zugeordnet, der wiederum einem Organ zugeordnet ist. Jede Spalte ist ferner in zwei Unterspalten eingeteilt, wobei die linke Unterspalte die Frequenzen angibt, die mit dem Anfang des entsprechenden Meridians verknüpft sind, während die rechte Teilspalte die Frequenzen angibt, die mit dem Ende des jeweiligen Meridians verknüpft sind. Die jeweiligen Anfangs- und Endfrequenzen eines Organs sind mit den jeweiligen Anfangs- und Endfrequenzen eines anderen Organs über ein besonderes Verhältnis verknüpft. Dieses Verhältnis entspricht dem Frequenzverhältnis der Töne einer Tonleiter einer Oktave. Die Frequenzen der Lunge entsprechen dabei dem Grundton einer Tonleiter. Zur Behandlung der jeweiligen Organe wird die entsprechende Frequenz an der ersten Frequenzgeneratoreinheit ausgegeben.

Die Zeilen sind jeweils ein $2^Z$-faches der Grundfrequenz, die hervorgehoben ist. Das heißt, eine Frequenz kann durch Multiplikation mit 2, 4, 8 usw. oder mit 1/2, 1/4, 1/8 usw. erreicht werden. Bevorzugt gibt die erste Frequenzgeneratoreinheit eine Wechselspannung mit der hervorgehobenen Frequenz aus, während die zweite und dritte Frequenzgeneratoreinheit jeweils eine Wechselspannung mit einer Frequenz ausgibt, die oberhalb oder unterhalb der Frequenz der ersten Frequenzgeneratoreinheit in einer Spalte angegeben ist.

Im Folgenden soll nun die Funktionsweise der Therapievorrichtung 10 erläutert werden.

Der Verwender der Therapievorrichtung steckt die Elektroden an der Therapievorrichtung 10 an den entsprechenden Anschlüssen 32, 34, 36 und 38 ein und bringt sie in Kontakt mit der Haut des Patienten. Danach stellt der Verwender mit Hilfe der Eingabeeinrichtung 46 die Frequenz, die Spannung und die Art der von der ersten Frequenzgeneratoreinheit 12 auszugebenden Spannung ein. Die Frequenz kann durch Eintippen einer Zahl eingegeben werden oder durch Eingeben einer Nummer, die in einem Speicher der Steuereinrichtung 18 mit einer bestimmten Frequenz verknüpft ist. Der Verwender orientiert sich beispielsweise bei der zu verwendenden Frequenz an der in Fig. 6 abgebildeten Tabelle. Diese Tabelle kann auch in der Steuereinrichtung 18 gespeichert sein, so dass der Verwender lediglich die Koordinaten der zu verwendenden Frequenz einstellt, das heißt, zum einen die Nummer des Organs oder Meridians und zum anderen den gewünschten Ober- oder Unterton eingibt. Anschließend variiert er die eingestellte Frequenz, um die verwendete Frequenz an den Patienten anzupassen.

Nun stellt der Verwender ein, ob die Frequenz die von der zweiten Frequenzgeneratoreinheit 14 und der dritten Frequenzgeneratoreinheit 16 ausgegebenen Wechselspannung das Zwei-, Vier-, Acht- usw. oder Einhalb-, Einviertel oder Einachtelfache der Frequenz der von der ersten Frequenzgeneratoreinheit 12 erzeugten Wechselspannung sein soll. Der Steuerungscomputer regelt dann die von der zweiten Frequenzgeneratoreinheit 14 oder der dritten Frequenzgeneratoreinheit 16 ausgegebene Frequenz selbstständig. Auch bei einer weiteren Änderung der Frequenz der von der ersten Frequenzgeneratoreinheit 12 ausgegebenen Wechselspannung werden automatisch die Frequenzen der anderen ausgegebenen Wechselspannungen geändert.

Nun stellt der Verwender die Spannung der von den Frequenzgeneratoreinheiten 12, 14 und 16 ausgegebenen Wechselspannungen ein. Besonders geeignet ist ein Verhältnis der Spannungen von 4 : 2 : 1. Alternativ kann für den Fall, dass eine Elektrode 39 an dem Neutralanschluss 38 angeschlossen ist, auch die Stromstärke eingestellt werden, die zwischen dem ersten Elektrodenausgang 26 und dem zweiten Elektrodenausgang 28 fließen soll. Die Steuereinrichtung 18 regelt dann die Spannung der Wechselspannung so, dass die eingestellte Stromstärke fließt.

In einem nächsten Schritt wird die Art der ausgegebenen Wechselspannung für die drei Frequenzgeneratoreinheiten 12, 14 und 16 eingestellt. Der Verwender kann zwischen einer Sinus-, Rechtecks- oder Dreiecksspannung wählen.

Daraufhin wird die Phasenverschiebung der drei ausgegebenen Wechselspannungen eingestellt.

Als letzter Schritt wird die Dauer der Behandlung an der Therapievorrichtung 10 eingestellt.

**Bezugszeichenliste**

10   Therapievorrichtung
12   erste Frequenzgeneratoreinheit
14   zweite Frequenzgeneratoreinheit
16   dritte Frequenzgeneratoreinheit
18   Steuereinrichtung
20   Energiespeicher
22   Gehäuse
24   Frequenzgenerator
26   erster Elektrodenausgang
28   zweiter Elektrodenausgang
30   Behälter
32   Handelektrodenanschluss
34   Abgreifelektrodenanschluss
36   BNC-Anschluss
38   Neutralanschluss
39   Elektrode
40   Spannungsausgang
42   Neutralausgang
43   Deckel
44   Anzeigeeinrichtung
46   Eingabeeinrichtung
48   Display
50   Behälterzugang
52   Klappe
54   Ladegerätanschluss

Die vorliegende Erfindung betrifft ferner ein Therapiegerät zur Behandlung von bevorzugt menschlichen Patienten. Insbesondere betrifft die Erfindung einen Zapper, bei dem über zwei Elektroden einem Patienten ein Wechselstrom zugeführt wird, der beispielsweise Parasiten im Körper des Patienten abtötet.

Ein solches Gerät ist aus der WO 2006/024150 A1 bekannt. Dabei handelt es sich um einen Frequenzgenerator, der über Elektroden mit den Händen oder Handgelenken eines Patienten verbunden wird. Anschließend kann eine vorausgewählte Frequenz über die Elektroden dem Patienten zugeführt werden. Damit kann ein Therapeut durch Auswahl von Behandlungsdauer, Stromstärke und Frequenz der angelegten Spannung eine individuell auf den Patienten abgestimmte Therapie durchführen.

Es hat sich jedoch gezeigt, dass die Bedienung eines solchen Multifrequenzgenerators einen hohen Aufwand darstellt und für manche Anwendungen nicht oder nur bedingt geeignet ist.

Es ist daher Aufgabe der vorliegenden Erfindung, ein Therapiegerät bereitzustellen, das bei einfacher und kostengünstiger Herstellung eine einfache Bedienung erlaubt.

Gelöst wird die Aufgabe durch die Merkmale des Anspruchs 21. Dieser offenbart ein Therapiegerät, das zumindest zwei, mit einem Patienten verbindbare Elektroden umfasst, von denen zumindest eine auch eine Erdungselektrode ist. Dabei werden die Elektroden von einer Wechselspannungsquelle mit einer Wechselspannung beaufschlagt, so dass die Wechselspannung von den Elektroden an den Patienten übertragen wird. Weiterhin umfasst das erfindungsgemäße Therapiegerät eine Steuervorrichtung, die mit der Wechselspannungsquelle verbunden ist und die eingerichtet ist, die Wechselspannungsquelle periodisch zu aktivieren und zu deaktivieren. Erfindungsgemäß ist vorgesehen, dass die Steuervorrichtung die Wechselspannungsquelle über die Dauer eines ersten Zeitraums, während dem die Erdungselektrode nicht geerdet ist, aktiviert und über die Dauer eines zweiten Zeitraums, während dem die Erdungselektrode geerdet ist, deaktiviert. Das erfindungsgemäße Therapiegerät senkt daher den Bedienaufwand für einen Benutzer, erlaubt einem Therapeuten dennoch eine flexible Gestaltung der Behandlung, wie es bereits bei den Geräten des Standes der Technik möglich ist.

Der Therapeut wird daher von Routineaufgaben entlastet und kann sich vorwiegend der Therapie des Patienten selbst widmen.

Die Unteransprüche haben bevorzugte Weiterbildungen der Erfindung zum Inhalt.

Das Therapiegerät weist eine Erdungselektrode auf. Die Erdungselektrode ist ebenfalls mit dem Patienten verbindbar und ermöglicht es, den Patienten zu erden. Es ist daher möglich, den Patienten zu entladen, wenn dieser durch das Therapiegerät oder durch andere Quellen, wie beispielsweise den Patienten umgebende statische elektrische Felder und/oder Magnetfelder, aufgeladen wurde.

Es ist vorgesehen, dass die Erdungselektrode mit der Steuervorrichtung verbunden ist. Die Steuervorrichtung kann die Erdung des Patienten aktivieren und deaktivieren, was gegengleich zum Aktivieren und Deaktivieren der Wechselspannungsquelle geschieht. So ist es vorteilhaft, die Erdungselektrode und damit den Patienten während des zweiten Zeitraums zu erden, während die Erdungselektrode und damit der Patient während des ersten Zeitraums nicht geerdet wird. Das Therapiegerät kann auf diese Weise sehr effektiv eingesetzt werden, da während des ersten Zeitraums der Patient mit dem Therapiegerät durch Anlegen einer Wechselspannung behandelt wird, während er während des zweiten Zeitraums zur Ruhe kommen und sämtliche aufgebrachte Ladung abfließen kann. Ein derartiges Therapieverfahren ist mit herkömmlichen Geräten nur sehr schwer durchzuführen, hat sich jedoch als sehr hilfreich erwiesen. Somit bietet diese Ausführungsform der Erfindung eine gezielte Entlastung des Therapeuten bei der Bedienung des Geräts, wodurch dieser eine bessere Therapie ausführen kann. Bevorzugt ist außerdem vorgesehen, dass die Erdungselektrode zumindest eine der Elektroden ist, über die die Wechselspannung an den Patienten angelegt wird. In diesem Fall werden lediglich zwei Elektroden benötigt, die abwechselnd eine Wechselspannung an den Patienten anlegen und den Patienten erden. Es ist daher vorteilhaft vorgesehen, dass über die Elektroden während des erstens Zeitraums eine Wechselspannung an den Patienten angelegt wird und dass zumindest eine Elektrode und damit der Patient während des zweiten Zeitraums geerdet ist.

Weiter ist vorteilhaft vorgesehen, dass das Therapiegerät ein Bedienelement umfasst, wobei das Bedienelement zumindest ein Eingabemittel aufweist. Das Bedienelement ist bevorzugt mit der Steuervorrichtung verbunden, so dass über das zumindest eine Eingabemittel die Länge des ersten Zeitraums und/oder die Länge des zweiten Zeitraums eingestellt werden kann. Der Therapeut behält daher volle Kontrolle über die Therapie und kann diese speziell auf jeden Patienten abstimmen. Jedoch entfällt die manuelle Bedienung des Therapiegeräts während einer laufenden Therapie.

Weiterhin ist bevorzugt vorgesehen, dass die Wechselspannungsquelle eingerichtet ist, eine sinusförmige oder eine rechteckförmige Wechselspannung zu erzeugen. Da ein Rechtecksignal durch dessen Zusammensetzung eine unendliche Anzahl von sinusförmigen Oberschwingungen umfasst, ist dieses sehr aggressiv und daher sehr gut geeignet, die Parasiten im Körper des Patienten abzutöten. Es kann auch vorteilhaft sein, wenn die Wechselspannungsquelle stets eine positive Spannung ausgibt, so dass die sinusförmige oder rechteckförmige Wechselspannung ein Offset enthält, das ein Absinken der Wechselspannung in den negativen Bereich verhindert. Durch dieses Offset wird das Therapiegerät besonders wirkungsvoll.

Die Wahl der Frequenz der Wechselspannung hat einen großen Einfluss auf die Wirksamkeit der Therapie und wird daher vom Therapeuten übernommen. Dieser wählt aus einem breiten Spektrum eine Frequenz aus, die optimal für den aktuellen Patienten geeignet ist. Es ist daher vorteilhaft vorgesehen, dass die Wechselspannungsquelle eingerichtet ist, eine Spannung mit einer Frequenz zwischen 1 Hz und 2 MHz zu erzeugen. Dieses erzeugbare Spektrum an Frequenzen ermöglicht die Verwendung des Therapiegeräts für eine breite Anwendung, so dass eine Vielzahl von unterschiedlichen Krankheiten bei einem Patienten behandelt werden können.

Bevorzugt ist weiterhin vorgesehen, dass der erste Zeitraum und der zweite Zeitraum gleich groß sind. Alternativ ist bevorzugt vorgesehen, dass der erste Zeitraum und der zweite Zeitraum unterschiedlich groß sind.

Es ist außerdem vorteilhaft, wenn eine Gesamttherapiedauer am Therapiegerät einstellbar ist. Die Steuervorrichtung ist daher bevorzugt eingerichtet, nach einer einstellbaren Gesamttherapiedauer die Wechselspannungsquelle zu deaktivieren. Besonders bevorzugt ist dabei vorgesehen, dass die Gesamtbehandlungsdauer ebenfalls über das vorteilhaft verwendbare Bedienelement einstellbar ist.

Schließlich ist es vorteilhaft, wenn der erste Zeitraum und/oder der zweite Zeitraum eine Dauer zwischen 1 und 30 Sekunden umfassen.

Weitere Einzelheiten, Merkmale und Vorteile ergeben sich aus der nachfolgenden Beschreibung und den Figuren. Es zeigen:

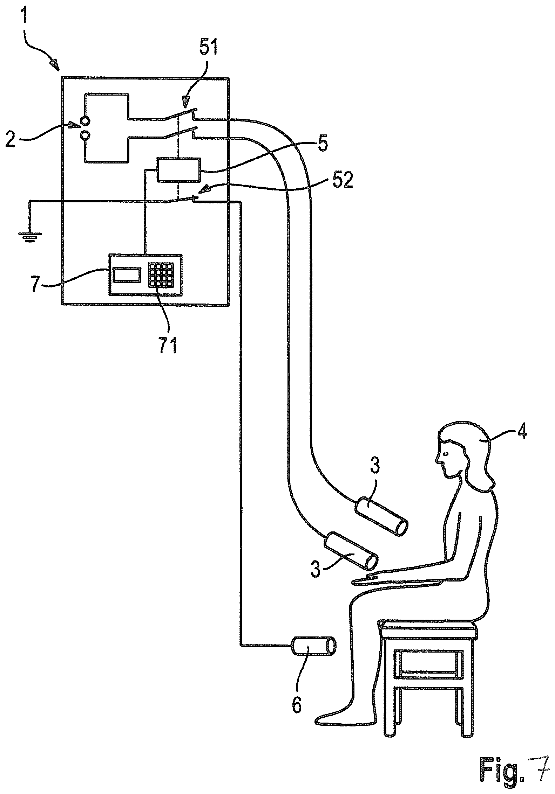

Fig. 7     eine schematische Übersicht über das Therapiegerät gemäß einem bevorzugten Ausführungsbeispiel der Erfindung, und

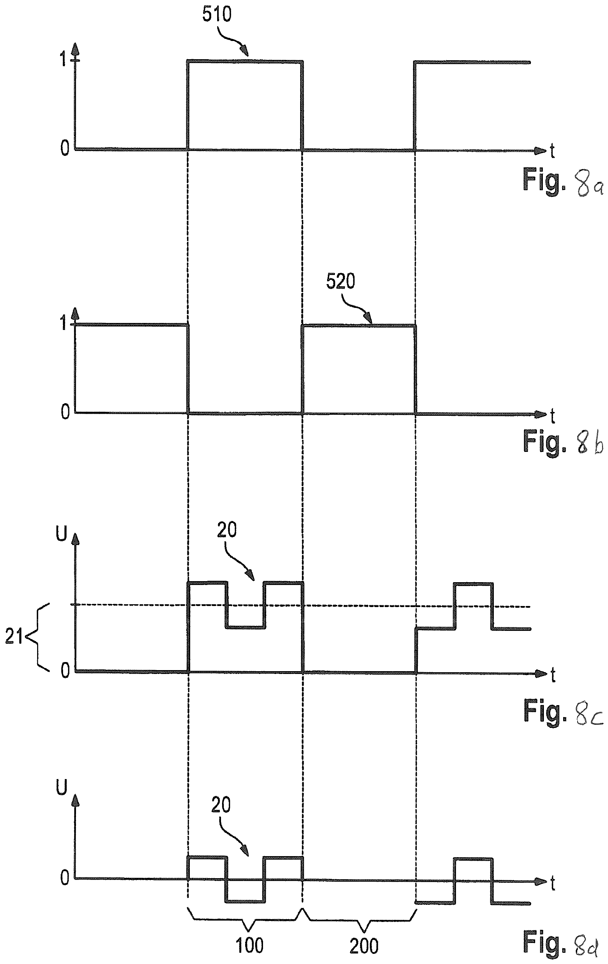

Fig. 8     eine schematische Darstellung über den logischen Aufbau des Therapiegeräts gemäß dem bevorzugten Ausführungsbeispiel der Erfindung.

Für die Beschreibung der Figuren 7 und 8 werden die in der folgenden Bezugszeichenliste aufgeführten Bezugszeichen verwendet.

Bezugszeichenliste

1     Therapiegerät

2     Wechselspannungsquelle

20     Spannungskurve

21     Positiver Offset in der Spannungskurve

3     Elektroden

| 4 | Patient |
|---|---|
| 5 | Steuervorrichtung |
| 51 | Erste Schaltvorrichtung |
| 510 | Erste Schaltkurve |
| 52 | Zweite Schaltvorrichtung |
| 520 | Zweite Schaltkurve |
| 6 | Erdungselektrode |
| 7 | Bedienelement |
| 71 | Eingabemittel |
| 100 | Erster Zeitraum |
| 200 | Zweiter Zeitraum |

Fig. 7 zeigt ein Therapiegerät 1, mit dem ein Patient 4 behandelt wird. Dazu umfasst das Therapiegerät 1 eine Spannungsquelle 2, die bevorzugt eine rechteckförmige Wechselspannung ausgibt. Die rechteckförmige Wechselspannung wird über eine erste Schaltvorrichtung 51 an zwei Elektroden 3 ausgegeben, die dazu verwendet werden, die rechteckförmige Wechselspannung an den Patienten 4 zu übertragen. Dazu kann der Patient 4 die Elektroden 3 beispielsweise in seine Hände nehmen. Alternativ können die Elektroden 3 auch über entsprechende Halterungen z. B. mit den Handgelenken des Patienten verbunden werden. Weiterhin ist eine Erdungselektrode 6 vorgesehen, die ebenfalls mit dem Patienten 4 verbindbar ist. Die Erdungselektrode 6 ist über eine zweite Schaltvorrichtung 52 geerdet.

Sowohl die erste Schaltvorrichtung 51 als auch die zweite Schaltvorrichtung 52 sind mit einer Steuervorrichtung 5 verbunden. Die Steuervorrichtung schaltet dabei die erste Schaltvorrichtung 51 und die zweite Schaltvorrichtung 52 gegengleich, so dass gleichzeitig mit einer Aktivierung der Elektroden 3 die Erdung der Erdungselektrode 6 deaktiviert wird. Werden die Elektroden 3 deaktiviert, so wird die Erdung der Erdungselektrode 6 aktiviert. Somit kann während eines ersten Zeitraums eine Spannung und ein Strom über die Elektroden 3 an den Patienten 4 übertragen werden, wobei anschließend während eines zweiten Zeitraums die Übertragung der Wechselspannung gestoppt und die Erdung über die Erdungselektrode 6 aktiviert wird, so dass eine verbleibende Aufladung des Patienten 4 abfließen kann.

Die Steuervorrichtung 5 ist weiter mit einem Bedienelement 7 verbunden, das ein Eingabemittel 71 aufweist. Über das Bedienelement 7 kann somit ein Therapeut die Parameter des Therapiegeräts 1 in die Steuervorrichtung 5 eingeben. Diese Parameter umfassen einerseits die Dauer des ersten Zeitraums, d. h. die Dauer der Aktivierung der Elektroden 3 und Deaktivierung der Erdungselektrode 6, den zweiten Zeitraum, d. h. die Deaktivierung der Elektroden 3 und die Aktivierung der Erdungselektrode 6, und einen Gesamtbehandlungszeitraum.

Die Fig. 8a, Fig. 8b, Fig. 8c und Fig. 8d verdeutlichen den logischen Aufbau des Therapiegeräts 1. Dabei zeigt Fig. 8a ein Diagramm mit einer ersten Schaltkurve, wobei die erste Schaltkurve 510 den Schaltungszustand der ersten Schaltvorrichtung 51 beschreibt. Fig. 8b zeigt ein Diagramm mit einer zweiten Schaltkurve 520, die den Schaltzustand der zweiten Schaltvorrichtung 52 beschreibt. Beide Kurven zeigen die Schalterstellungen der ersten Schaltvorrichtung 51 bzw. der zweiten Schaltvorrichtung 52 als logische Darstellung, so dass eine logische "1" einen eingeschalteten Zustand und eine logische "0" einen ausgeschalteten Zustand darstellt.

Schließlich zeigen die Fig. 8c und Fig. 8d eine Spannungskurve 20, die den Verlauf der Wechselspannung beschreibt, die an den Elektroden 3 anliegt. Fig. 8c zeigt eine Wechselspannung mit positivem Offset 21, während Fig. 8d einen alternativen Wechselspannungsverlauf ohne Offset zeigt.

Beispielhaft wird ein Zyklus, umfassend einen ersten Zeitraum 100 und einen zweiten Zeitraum 200, betrachtet. Zu Beginn des ersten Zeitraums 100 wird die erste Schaltvorrichtung 51 von der Steuervorrichtung 5 aktiviert. Aus diesem Grund springt die erste Schaltkurve 510 aus der Nulllage auf die logische "1". Gleichzeitig wird jedoch die zweite Schaltvorrichtung 52 deaktiviert, so dass die zweite Schaltkurve 520 von der logischen "1" auf die Nulllage zurückfällt. Somit ist während des ersten Zeitraums 100 eine Erdung des Patienten 4 deaktiviert, während die Elektroden 3 eine Spannung an den Patienten 4 abgeben. Dies ist ersichtlich, da die Spannungskurve 20 eine rechteckförmige Wechselspannung (in Fig. 8c mit positivem Offset 21, in Fig. 8d ohne Offset) mit vordefinierter Frequenz anzeigt.

An den ersten Zeitraum 100 schließt sich ein zweiter Zeitraum 200 an. Zu Beginn des zweiten Zeitraums 200 wird nun die erste Schaltvorrichtung 51 durch die Steuervorrichtung 5 deaktiviert, während die zweite Schaltvorrichtung 52 durch die Steuervorrichtung 5 aktiviert wird. Somit fällt die erste Schaltkurve 510 wieder auf die Nulllage zurück, während die zweite Schaltkurve 520 auf die logische "1" springt. Während des zweiten Zeitraums 200 ist somit eine Erdung des Patienten 4 aktiv, während die Elektroden 3 keine Spannung an den Patienten 4 übertragen. Daher fällt auch die Spannungskurve 20 in die Nulllage zurück.

Nach Verstreichen des zweiten Zeitraums 200 beginnt der Zyklus erneut, so dass wiederum für die Zeitdauer des ersten Zeitraums 100 die erste Schaltvorrichtung 51 aktiviert und die zweite Schaltvorrichtung 52 deaktiviert wird.

Nach einer vom Therapeuten eingestellten Behandlungsdauer wird die erste Schaltvorrichtung 51 deaktiviert. Die Behandlung des Patienten ist damit beendet. Es entsteht daher für den Therapeuten nur noch ein sehr geringer Bedienaufwand der erfindungsgemäßen Therapievorrichtung 1.

Die Erfindung bezieht sich außerdem auf eine Therapievorrichtung zur Behandlung mit Schwingungen.

Aus dem Stand der Technik ist es bekannt, bioenergetische Schwingungen zur Therapie einzusetzen, um eine elektrophysikalische Behandlung eines lebenden Organismus durchzuführen. Hierzu zeigt der Stand der Technik beispielsweise in der DE 297 09 094 U1 ein Gerät für die Bioresonanz-Therapie. Bei diesem Gerät wird der Körper durch Schwingungen in Verbindung mit schwachen Strömen durchströmt, wobei eine elektrische Stimulation hervorgerufen wird. Um den Fluss von Strom durch den Körper des Patienten zu vermeiden wird in DE 20 2010 010 700 U1 eine Therapievorrichtung offenbart, bei deren Anwendung der Patient Schwingungen in eine geerdete Antenne abstrahlt. Es hat sich jedoch gezeigt, dass die aus dem Stand der Technik bekannten Vorrichtungen nicht für alle Therapiefälle wirkungsvoll einsetzbar sind.

Der Erfindung liegt die Aufgabe zugrunde, eine Therapievorrichtung zu schaffen, welche bei einfachem Aufbau und einfacher, kostengünstiger Herstellbarkeit eine gute Therapierbarkeit eines lebenden Organismus ermöglicht.

Erfindungsgemäß wird die Aufgabe durch die Merkmalskombination des Anspruchs 31 gelöst, die Unteransprüche zeigen weitere vorteilhafte Ausgestaltungen der Erfindung.

Erfindungsgemäß ist somit zumindest ein Frequenzgenerator zur Abgabe von elektromagnetischen Schwingungen an einen Patienten vorgesehen. Hierzu weist der Frequenzgenerator eine mit diesem bevorzugt über ein Kabel verbundene Elektrode auf, welche an einem Patienten angeordnet werden kann, beispielsweise durch Auflegen auf seine Haut.

Durch die von dem Frequenzgenerator abgegebene Frequenz wird der Organismus des zu therapierenden Patienten erregt und strahlt die als Resonanz hervorgerufene Schwingung ab.

Erfindungsgemäß ist der Frequenzgenerator ausgebildet, zumindest zwölf festgelegte, unterschiedliche erste Frequenzen zu erzeugen. Diese zwölf Frequenzen liegen alle in innerhalb eines ersten Bereichs, dessen Grenzen bevorzugt einem Oktavengesetz folgen. So ist die untere Grenze gleich der niedrigsten Frequenz der zwölf ersten Frequenzen. Die obere Grenze ist durch die doppelte Frequenz der unteren Grenze gebildet, so dass sich ein oktavenspezifisches Verhältnis von 2:1 ergibt. Erfindungsgemäß ist die obere Grenze aus dem ersten Bereich ausgeschlossen.

Eine derartige Therapievorrichtung stellt die Grundlage für eine effektive Behandlung eines Patienten dar. Beispielsweise können die zwölf Frequenzen den zwölf Hauptmeridianen der Traditionellen Chinesischen Medizin zugeordnet werden, wodurch es dem Behandelnden Therapeuten vereinfacht wird, diese Meridiane mit entsprechenden Frequenzen zu aktivieren.

In einer bevorzugten Ausführungsform der Erfindung ist der Frequenzgenerator ausgebildet, die ersten Frequenzen in konstanten Verhältnissen zueinander zu erzeugen. Die Verhältnisse müssen dabei nicht zwangsläufig identisch sein, sind jedoch bevorzugt konstant, so dass bei Variation einer der zwölf ersten Frequenzen sämtliche weiteren Frequenzen der ersten Frequenzen gemäß der bevorzugten Verhältnissen ebenso geändert werden.

Weiterhin sind die Verhältnisse der ersten Frequenzen untereinander derart ausgebildet, dass sie gleich zu den Verhältnissen sind, die die zwölf Halbtöne einer Oktave der Klanglehre aufweisen. Durch diese Gesetzmäßigkeiten ist es möglich, eine "Tonleiter" der ersten Frequenzen aufzustellen, die der Tonleiter einer Oktave mit 12 Halbtönen gleicht.

Weiterhin ist erfindungsgemäß bevorzugt vorgesehen, dass die ersten Frequenzen zueinander ein Verhältnis aufweisen, das identisch mit einem Halbton und/oder einem Viertelton aus der Klanglehre ist. Die erfindungsgemäße Therapievorrichtung kann daher diskrete Frequenzwerte abgeben, so dass dem Therapeuten die Auswahl der für den aktuellen Patienten passenden Frequenz erleichtert wird.

In einer weiteren vorteilhaften Ausführungsform der Erfindung ist der Frequenzgenerator ausgebildet, neben den zwölf ersten Frequenzen weitere Frequenzen zu erzeugen. Bevorzugt folgen die weiteren Frequenzen denselben Gesetzmäßigkeiten wie die ersten Frequenzen. Somit liegen die weiteren Frequenzen innerhalb eines weiteren Bereichs, der durch die niedrigste der weiteren Frequenzen nach unten begrenzt wird. Weiterhin ist auch für jeden weiteren Bereich die Obergrenze als doppelte Frequenz der Frequenz der Untergrenze definiert. Auf diese Weise sind die Voraussetzungen vorhanden, die weiteren Frequenzen analog zu aus der Klanglehre bekannten Oktaven um den ersten Bereich herum anzuordnen. Jede weitere Frequenz steht somit in einem festen Verhältnis zu einer der ersten Frequenzen, wobei die jeweils größere Frequenz um ein natürliches Vielfaches von 2 größer als die jeweils kleinere Frequenz ist. Dadurch ergibt sich ein aus der Klanglehre bekanntes Bild, in dem der gesamte Frequenzraum in eine Aneinanderreihung von oktavenähnlichen Bereichen aufgeteilt ist.

Eine derartige Oktavierung der möglichen Frequenzen, mit denen ein Patient behandelt werden kann, macht es für den Therapeuten deutlich einfacher, eine zu verwendende Frequenz für die Behandlung zu finden. So kann beispielsweise eine Frequenz, die einem bestimmten Meridian zugeordnet ist, als Ton angesehen werden, der jedoch auch in anderen Oktaven vorkommt. Dies bedeutet, dass beispielsweise eine doppelt oder halb so große Frequenz auf denselben Meridian wirkt. Dem Therapeuten steht somit offen, eine bestmöglichste Frequenz gemäß dem Oktavengesetz auszuwählen.

Erfindungsgemäß ist die obere Grenze und/oder die untere Grenze des ersten Bereichs bevorzugt manuell anpassbar. Alternativ oder zusätzlich ist die obere Grenze und/oder die untere Grenze des ersten Bereichs bevorzugt mittels einer automatischen Steuereinheit einstellbar.

Die Anpassbarkeit der Grenzen erlaubt eine vorteilhafte Kalibrierung der Therapievorrichtung auf einen bestimmten Patienten. Da die ersten Frequenzen nicht bei allen Patienten exakt gleich sein müssen, kann hier der Therapeut korrigierend eingreifen und die ersten Frequenzen und damit den ersten Bereich entsprechend anpassen. Da die Verhältnisse der Frequenzen untereinander bevorzugt konstant sind, verschiebt sich somit lediglich die Frequenzlage als ganzes, während die grundsätzliche Oktavierung erhalten bleibt.

In besonders günstiger Weiterbildung der Erfindung ist vorgesehen, dass zumindest drei Frequenzgeneratoren verwendet werden. Diese können beispielsweise über jeweils separate Kabel und Elektroden mit dem Patienten verbunden sein. In vorteilhafter Weise können die drei Frequenzgeneratoren dazu eingesetzt werden, die gleichen Frequenzen zu erzeugen, um die gewünschte Therapiewirkung zu potenzieren.

Alternativ können die drei Frequenzgeneratoren verwendet werden, um unterschiedliche Frequenzen zu erzeugen. Dies ermöglicht beispielsweise die Erzeugung eines Dreiklangs mit drei Frequenzen, die jeweils die gleiche Position innerhalb unterschiedlicher Oktaven einnehmen, was bedeutet, dass diese in einem Verhältnis stehen, das ein Vielfaches von 2 ist. Auch dies kann die Wirkung der Therapie auf den Patienten verbessern.

Alternativ oder zusätzlich können die Betriebsspannungen der Frequenzgeneratoren gleich oder voneinander verschieden gewählt sein. Hier ergibt sich für den Therapeuten eine weitere vorteilhafte Einstellmöglichkeit, wie der Therapieerfolg verbessert werden kann.

In besonders günstiger Ausgestaltung der Erfindung ist vorgesehen, dass im Bereich des Kabels zwischen dem Frequenzgenerator und der Elektrode ein Behälter zur Aufnahme einer Substanz oder einer Probe angeordnet ist. Dieser Behälter kann beispielsweise in Form eines Bechers ausgebildet sein, in welchen chemische Substanzen, Nosoden, Organproben oder Ähnliches eingebracht sind. Diese modulieren die Frequenz des Frequenzgenerators. Bei Verwendung mehrerer Frequenzgeneratoren mit mehreren derartigen Behältern ist es beispielsweise möglich, unterschiedliche Organe des Patienten zu therapieren oder unterschiedliche Belastungen des Patienten zu berücksichtigen, beispielsweise zur Behandlung eines inneren Organs und zur Ausleitung von Giften aus dem Körper des Patienten.

Erfindungsgemäß ist es besonders günstig, wenn der Frequenzgenerator Sinuswellen, Rechteckwellen oder beliebige andere Wellen erzeugt, die beispielsweise in Frequenzen von 0,01 Hz bis 40 MHz liegen. Dabei werden Spannungen von $\pm$ 0,1 V bis $\pm$ 20 V angelegt.

Es ist erfindungsgemäß auch in günstiger Ausgestaltung möglich, einen Frequenzgenerator als Wobbelgenerator auszubilden. Derartige Wobbelgeneratoren oder Wobbler erzeugen eine Ausgangsfrequenz durch Mischung verschiedener Frequenzen, wobei durch Programmsteuerung eine Wobbelung realisiert werden kann, beispielsweise in Form eines Sägezahn- oder Rechteckprofils.

Diese als Resonanz auftretende Strahlung oder Erregung des Organismus kann bevorzugt von einer Antenne aufgenommen werden, welche die elektromagnetischen Schwingungen aufnimmt und erdet. Die Antenne ist erfindungsgemäß in besonders vorteilhafter Weise in Form einer Teslaantenne ausgebildet, welche in besonders günstiger Weise die vom Körper des Patienten gesendeten Frequenzbereiche oder Frequenzen aufnimmt oder erdet.

Die bevorzugt vorgesehene Antenne ist bevorzugterweise in einem Abstand von 20 bis 50 cm vom zu therapierenden Patienten angeordnet. Der Patient reagiert somit auf die Erregung durch die aufgebrachten Frequenzen und sendet ein ihm eigenes Frequenzmuster ab, welches durch die Antenne aufgenommen und geerdet wird.

Im Folgenden wird die Erfindung anhand eines Ausführungsbeispiels in Verbindung mit der Zeichnung beschrieben. Dabei zeigt:

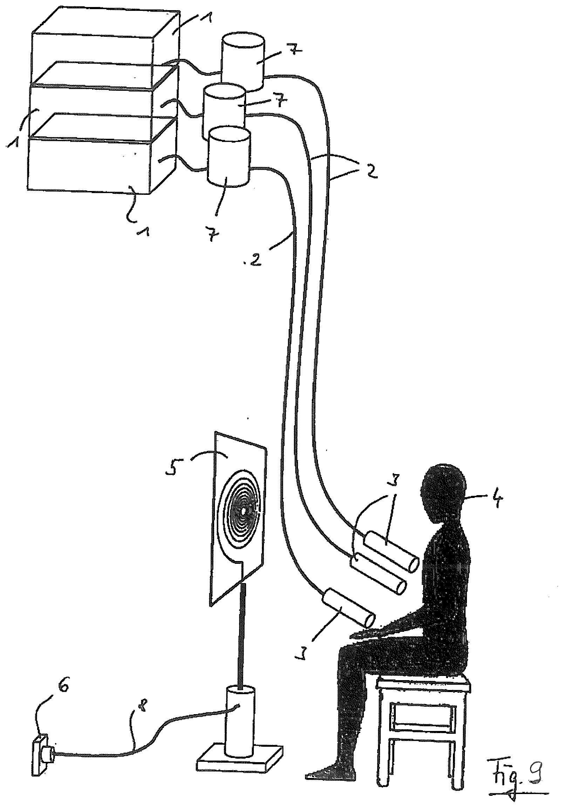

Fig. 9 eine schematische Darstellung der erfindungsgemäßen Therapievorrichtung.

Fig. 9 wird anhand der in der folgenden Bezugszeichenliste aufgeführten Bezugszeichen beschrieben.

Bezugszeichenliste

1     Frequenzgenerator

2     Kabel

3     Elektrode

4     Patient

5     Antenne

6     Erdung

7     Behälter

8     Kabel

Das Ausführungsbeispiel der Fig. 9 zeigt drei Frequenzgeneratoren 1, welche über Kabel 2 mit Elektroden 3 verbunden sind. Die Kabel 2 sind als flexible elektrisch leitende Kabel ausgebildet, die Elektroden 3 sind aus Metall gefertigt oder haben zumindest metallische Kontaktflächen oder Kontaktflächen aus anderen leitenden Materialien, um an die Haut des Patienten angelegt werden zu können, so dass die von den Frequenzgeneratoren abgegebenen Frequenzen (Schwingungen) auf den Körper des Patienten übertragbar sind.

In den Kabeln 2 sind jeweils Behälter 7 zwischengeschaltet, welche beispielsweise als Becher ausgebildet sein können, um Arzneimittel, Proben, chemische Stoffe oder Ähnliches aufzunehmen.

In einer besonders vorteilhaften Anwendung des Therapiegerät aber nicht erfindungsgemäß vorgesehen ist in direkter Zuordnung zu dem Patienten 4 eine Antenne 5 angeordnet, welche bevorzugterweise als Teslaantenne ausgebildet ist und über ein Kabel 8 mit einer Erdung 6 verbunden ist. Die Antenne 5 kann, wie in Fig. 9 gezeigt, als singuläre Antenne ausgebildet sein, es ist jedoch auch möglich, diese räumlich um den Patienten 4 anzuordnen. Die Teslaantenne weist jeweils zumindest eine rechtsdrehende und eine linksdrehende Spule auf.

Der Frequenzgenerator (1) kann an einen Patienten (4) beispielsweise eine Frequenz von 68,72 Hz abgeben, wodurch sich positive Effekte auf die Lunge ergeben können. Erfindungsgemäß bestimmt die Therapievorrichtung daraus weitere Frequenzen, wie beispielsweise 77,31 Hz, die positive Effekte auf den Magen ergeben kann. Dabei stehen diese Frequenzen in einem Verhältnis von 9/8, was dem Verhältnis d': c' der chromatischen Tonleiter entspricht. Eine weitere Frequenz von 82,46 Hz kann sich positiv auf die Milz auswirken. Diese Frequenz steht zur Frequenz der Lunge im Verhältnis 6/5, was dem Verhältnis es': c' der chromatischen Tonleiter entspricht.

Die Therapievorrichtung ist dabei zusätzlich eingerichtet, dass ein Therapeut diese Werte verändern kann. So kann beispielsweise die Frequenz der Lunge abweichend 68,70 Hz betragen. Sobald diese manuell angepasst wird, werden sämtliche anderen Frequenzen gemäß der beispielhaft genannten Verhältnisse aktualisiert. Somit steht für jeden individuellen Organismus eine optimale Oktavierung der Frequenzen zur Verfügung.

Damit ermöglicht es die erfindungegemäße Therapievorrichtung, einen Therapeuten effizient zu unterstützen. Dieser wählt eine auf den Patienten (4) und eine auf den zu behandelnden Meridian aus der Traditionellen Chinesischen Medizin abgestimmte Frequenz, wobei ihm die Therapievorrichtung sämtliche optimalen Frequenzen für die zwölf Meridiane des menschlichen Körpers innerhalb einer Oktave zur Verfügung stellt. Da diese Frequenzen den Halbtonschritten einer aus der Musik bekannten Oktave entsprechen, können dieselben Meridiane ebenso mit einer höheren oder tieferen Frequenz behandelt werden, wenn ein Oktavenverhältnis von 2:1 eingehalten wird. Auch hier werden dem Therapeuten die passenden Frequenzen von der Therapievorrichtung zur Verfügung gestellt, so dass er wirkungsvoll in Anwendung seiner Heilkunst unterstützt wird.

## Patentansprüche

1. Therapiegerät (1) zur Behandlung von bevorzugt menschlichen Patienten (4), umfassend:

   zumindest zwei, mit einem Patienten (4) verbindbare Elektroden (3, 6), von denen zumindest eine auch eine Erdungselektrode (6) ist;
   eine Wechselspannungsquelle (2), die eine Wechselspannung an die zwei Elektroden (3) anlegt, und
   eine Steuervorrichtung (5), die mit der Wechselspannungsquelle (2) verbunden ist;
   wobei die Steuervorrichtung (5) eingerichtet ist, die Wechselspannungsquelle (2) periodisch für einen ersten Zeitraum (100), während dem die Erdungselektrode (6) nicht geerdet ist, zu aktivieren, so dass die Elektroden (3, 6) mit einer Wechselspannung beaufschlagt werden, die an den Patienten (4) zur Behandlung übertragbar ist;
   wobei die Steuervorrichtung (5) ferner eingerichtet ist, die Wechselspannungsquelle (2) periodisch für einen zweiten Zeitraum (200), während dem die Erdungselektrode (6) geerdet ist, zu deaktivieren, um Ladung von dem Patienten (4) abfließen zu lassen.

2. Therapiegerät (1) nach Anspruch 1, **gekennzeichnet durch** ein Bedienelement (7), wobei das Bedienelement (7) mit der Steuervorrichtung (5) verbunden ist und zumindest ein Eingabemittel (71) aufweist, über das die Länge des ersten Zeitraums (100) und/oder die Länge des zweiten Zeitraums (200) eingestellt werden kann.

3. Therapiegerät (1) nach Anspruch 1 oder 2, **dadurch gekennzeichnet, dass** die Wechselspannungsquelle (2) eingerichtet ist, eine sinusförmige oder eine rechteckförmige Wechselspannung zu erzeugen, wobei vorzugsweise die Wechselspannung einen positiven Offset (21) aufweist.

4. Therapiegerät (1) nach einem der Ansprüche 1 bis 3, **dadurch gekennzeichnet, dass** die Wechselspannungsquelle (2) eingerichtet ist, eine Spannung mit einer Frequenz zwischen 1 Hz und 2 MHz zu erzeugen.

5. Therapiegerät nach einem der Ansprüche 1 bis 4, **dadurch gekennzeichnet, dass** der erste Zeitraum (100) und der zweite Zeitraum (200) unterschiedlich groß sind.

6. Therapiegerät nach einem der Ansprüche 1 bis 5, **dadurch gekennzeichnet, dass** die Steuervorrichtung (5) eingerichtet ist, die Wechselspannungsquelle (2) nach dem Verstreichen einer Gesamtbehandlungsdauer zu deaktivieren.

**7.** Therapiegerät nach einem der Ansprüche 1 bis 6, **dadurch gekennzeichnet, dass** der erste Zeitraum (100) und/oder der zweite Zeitraum (200) eine Dauer zwischen 1 und 30 Sekunden umfassen.

**8.** Therapiegerät nach einem der Ansprüche 1 bis 7, **dadurch gekennzeichnet, dass** die Elektroden (3, 6) so ausgestaltet sind, dass der Patient (4) diese in seine Hände nehmen kann.

**9.** Therapiegerät nach einem der Ansprüche 1 bis 7, **dadurch gekennzeichnet, dass** Elektroden (3, 6) über Halterungen mit den Handgelenken des Patienten (4) verbindbar sind.

**10.** Therapiegerät nach einem der Ansprüche 1 bis 9, **gekennzeichnet durch** einen erste Schaltvorrichtung (51) und eine zweite Schaltvorrichtung (52), die mit der Steuervorrichtung (5) verbunden sind.

**11.** Therapiegerät nach Anspruch 10, **dadurch gekennzeichnet, dass** die Steuervorrichtung (5) eingerichtet ist, die erste Schaltvorrichtung (51) und die zweite Schaltvorrichtung (52) gegengleich zu schalten, so dass während des ersten Zeitraums (100) eine Wechselspannung über die Elektroden (3, 6) an den Patienten (4) übertragbar ist und während des zweiten Zeitraums (200) die Übertragung der Wechselspannung unterbrochen und wenigstens eine der Elektroden (3, 6) geerdet ist.

**12.** Therapiegerät nach Anspruch 10 oder 11, **gekennzeichnet durch** eine erste Schaltkurve (510) für die erste Schaltvorrichtung (51) und eine zweite Schaltkurve (520) für die zweite Schaltvorrichtung (52), wobei vorzugsweise die erste Schaltkurve (51) während des ersten Zeitraums (100) eine logische 1 und während des zweiten Zeitraums (200) eine Null aufweist und wobei weiter vorzugsweise die zweite Schaltkurve (52) während des ersten Zeitraums (100) eine Null und während des zweiten Zeitraums (200) eine logische 1 aufweist.

**Claims**

**1.** Therapy device (1) for treating preferably human patients (4), comprising:

at least two electrodes (3, 6) connectable to a patient (4) with at least one of which being a grounding electrode (6);
an alternating power source (2) applying an alternating voltage to the two electrodes (3) and
a control device (5) connected to the alternating power source (2);

wherein the control device (5) is configured to activate the alternating voltage source (2) periodically for a first time period (100) while the grounding electrode (6) is not grounded so that the electrodes (3, 6) are supplied with an alternating voltage being transferable to the patient (4) for treatment;
wherein the control device (5) is further configured to periodically deactivate the alternating voltage source (2) for a second time period (200) while the grounding electrode (6) is grounded so that charge can be drained off the patient (4).

**2.** Therapy device (1) according to claim 1, **characterised by** an operating element (7), wherein the operating element (7) is connected to the control device (5) and has at least one input means (71) via which the length of the first time period (100) and/or the length of the second time period (200) can be set.

**3.** Therapy device (1) according to claim 1 or 2, **characterised in that** the alternating voltage source (2) is configured to generate a sinusoidal or a rectangular alternating voltage, wherein preferably the alternating voltage has a positive offset (21).

**4.** Therapy device (1) according to one of the claims 1 to 3, **characterised in that** the alternating voltage source (2) is configured to generate a voltage having a frequency between 1 Hz and 2 MHz.

**5.** Therapy device according to one of the claims 1 to 4, **characterised in that** the first time period (100) and the second time period (200) are of different durations.

**6.** Therapy device according to one of the claims 1 to 5, **characterised in that** the control device (5) is configured to deactivate the alternating voltage source (2) after the lapse of a total treatment period.

**7.** Therapy device according to one of the claims 1 to 6, **characterised in that** the first time period (100) and/or the second time period (200) comprise a duration between 1 and 30 seconds.

**8.** Therapy device according to one of the claims 1 to 7, **characterised in that** the electrodes (3, 6) are designed so that the patient (4) can take these in his hands.

**9.** Therapy device according to one of the claims 1 to 7, **characterised in that** electrodes (3, 6) are connectable via mounting brackets with the wrists of the patient (4).

**10.** Therapy device according to one of the claims 1 to 9, **characterised by** a first switching device (51) and a second switching device (52), which are connected to the control device (5).

11. Therapy device according to claim 10, **characterised in that** the control device (5) is configured to switch counter to each other the first switching device (51) and the second switching device (52), so that during the first time period (100) an alternating voltage is transferable via the electrodes (3 , 6) to the patient (4) and during the second time period (200) the transfer of the alternating voltage is interrupted and at least one of the electrodes (3, 6) is grounded.

12. Therapy device according to claim 10 or 11, **characterised by** a first switching curve (510) for the first switching device (51) and a second switching curve (520) for the second switching device (52), wherein preferably the first switching curve (51) during the first period (100) has a logical 1 and during the second time period (200) a zero, and wherein further preferably the second switching curve (52) has a zero during the first time period (100) and a logical 1 during the second time period (200).

**Revendications**

1. Dispositif thérapeutique (1) pour le traitement de patients, de préférence humains (4), comprenant :

   au moins deux électrodes (3, 6) pouvant être connectées à un patient (4), dont l'une au moins est également une électrode de mise à la terre (6) ;
   une source de tension alternative (2) qui applique une tension alternative aux deux électrodes (3), et
   un dispositif de commande (5) connecté à la source de tension alternative (2) ;
   dans lequel
   le dispositif de commande (5) est conçu pour activer périodiquement la source de tension alternative (2) pendant une première période de temps (100) pendant laquelle l'électrode de mise à la terre (6) n'est pas mise à la terre, de sorte que les électrodes (3, 6) sont alimentées avec une tension alternative qui peut être transmise au patient (4) pour traitement ;
   le dispositif de commande (5) est en outre conçu pour désactiver périodiquement la source de tension alternative pendant une seconde période de temps (200) pendant laquelle l'électrode de mise à la terre (6) est mise à la terre, pour évacuer la charge depuis le patient (4).

2. Dispositif thérapeutique (1) selon la revendication 1, **caractérisé par** un élément de manipulation (7), l'élément de manipulation (7) étant connecté au dispositif de commande (5) et comprenant au moins un moyen de saisie (71) permettant de régler la longueur de la première période de temps (100) et/ou la longueur de la seconde période de temps (200).

3. Dispositif thérapeutique (1) selon la revendication 1 ou 2, **caractérisé en ce que** la source de tension alternative (2) est conçue pour générer une tension alternative sinusoïdale ou rectangulaire, la tension alternative présentant de préférence un décalage (offset) positif (21).

4. Dispositif thérapeutique (1) selon l'une des revendications 1 à 3, **caractérisé en ce que** la source de tension alternative (2) est conçue pour générer une tension à une fréquence entre 1 Hz et 2 MHz.

5. Dispositif thérapeutique selon l'une des revendications 1 à 4, **caractérisé en ce que** la première période de temps (100) et la seconde période de temps (200) ont différentes longueurs.

6. Dispositif thérapeutique selon l'une des revendications 1 à 5, **caractérisé en ce que** le dispositif de commande (5) est conçu pour désactiver la source de tension alternative (2) après expiration d'une durée de traitement totale.

7. Dispositif thérapeutique selon l'une des revendications 1 à 6, **caractérisé en ce que** la première période de temps (100) et/ou la seconde période de temps (200) ont une durée entre 1 et 30 secondes.

8. Dispositif thérapeutique selon l'une des revendications 1 à 7, **caractérisé en ce que** les électrodes (3, 6) sont conçues de telle sorte que le patient (4) peut les prendre dans ses mains.

9. Dispositif thérapeutique selon l'une des revendications 1 à 7, **caractérisé en ce que** les électrodes (3, 6) peuvent être reliées aux poignets du patient (4) par des éléments de maintien.

10. Dispositif thérapeutique selon l'une des revendications 1 à 9, **caractérisé par** un premier dispositif de commutation (51) et par un second dispositif de commutation (52) qui sont connectés au dispositif de commande (5).

11. Dispositif thérapeutique selon la revendication 10, **caractérisé en ce que** le dispositif de commande (5) est conçu pour commuter en sens opposés le premier dispositif de commutation (51) et le second dispositif de commutation (52), de sorte que pendant la première période de temps (100), une tension alternative peut être transmise au patient (4) via les électrodes (3, 6), et que pendant la seconde période de temps (200) la transmission de la tension alternative est interrompue et l'une au moins des électrodes (3, 6) est mise à la terre.

12. Dispositif thérapeutique selon la revendication 10 ou 11, **caractérisé par** une première courbe de commutation (510) pour le premier dispositif de commutation (51) et par une seconde courbe de commutation (520) pour le second dispositif de commutation (52), et de préférence la première courbe de commutation (51) présente un 1 logique pendant la première période de temps (100) et présente un zéro pendant la seconde période de temps (200), et en outre, de préférence, la seconde courbe de commutation présente un zéro pendant la première période de temps (100) et présente un 1 logique pendant la seconde période de temps (200).

Fig. 1

Fig. 2

Fig. 3

Fig. 4

Fig. 5

EP 2 944 350 B1

| Lunge A. | Lunge E. | Dickdarm A. | Dickdarm E. | Magen A. | Magen E. | MiPa A. | MiPa E. | Herz A. | Herz E. |
|---|---|---|---|---|---|---|---|---|---|
| 0.033554688 | 0.0356934 | 0.036135742 | 0.03838623 | 0.037749 | 0.0401001 | 0.0402656 | 0.0427734 | 0.0419434 | 0.044555664 |
| 0.067109375 | 0.0713867 | 0.072271484 | 0.076772461 | 0.075498 | 0.0802002 | 0.0805313 | 0.0855469 | 0.0838867 | 0.089111328 |
| 0.13421875 | 0.1427734 | 0.144542969 | 0.153544922 | 0.1509961 | 0.1604004 | 0.1610625 | 0.1710938 | 0.1677734 | 0.178222656 |
| 0.2684375 | 0.2855469 | 0.289085938 | 0.307089844 | 0.3019922 | 0.3208008 | 0.322125 | 0.3421875 | 0.3355469 | 0.356445313 |
| 0.536875 | 0.5710938 | 0.578171875 | 0.614179688 | 0.6039844 | 0.6416016 | 0.64425 | 0.684375 | 0.6710938 | 0.712890625 |
| 1.07375 | 1.1421875 | 1.15634375 | 1.228359375 | 1.2079688 | 1.2832031 | 1.2885 | 1.36875 | 1.3421875 | 1.42578125 |
| 2.1475 | 2.284375 | 2.3126875 | 2.45671875 | 2.4159375 | 2.5664063 | 2.577 | 2.7375 | 2.684375 | 2.8515625 |
| 4.295 | 4.56875 | 4.625375 | 4.9134375 | 4.831875 | 5.1328125 | 5.154 | 5.475 | 5.36875 | 5.703125 |
| 8.59 | 9.1375 | 9.25075 | 9.826875 | 9.66375 | 10.265625 | 10.308 | 10.95 | 10.7375 | 11.40625 |
| 17.18 | 18.275 | 18.5015 | 19.65375 | 19.3275 | 20.53125 | 20.616 | 21.9 | 21.475 | 22.8125 |
| 34.36 | 36.55 | 37.003 | 39.3075 | 38.655 | 41.0625 | 41.232 | 43.8 | 42.95 | 45.625 |
| 68.72 | 73.1 | 74.006 | 78.615 | 77.31 | 82.125 | 82.464 | 87.6 | 85.9 | 91.25 |
| 137.44 | 146.2 | 148.012 | 157.23 | 154.62 | 164.25 | 164.928 | 175.2 | 171.8 | 182.5 |
| 274.88 | 292.4 | 296.024 | 314.46 | 309.24 | 328.5 | 329.856 | 350.4 | 343.6 | 365 |
| 549.76 | 584.8 | 592.048 | 628.92 | 618.48 | 657 | 659.712 | 700.8 | 687.2 | 730 |
| 1099.52 | 1169.6 | 1084.096 | 1257.84 | 1236.96 | 1314 | 1319.424 | 1401.6 | 1374.4 | 1460 |
| 2199.04 | 2339.2 | 2368.192 | 2515.68 | 2473.92 | 2628 | 2638.848 | 2803.2 | 2748.8 | 2920 |
| 4398.08 | 4678.4 | 4736.384 | 5031.36 | 4947.84 | 5256 | 5277.696 | 5606.4 | 5497.6 | 5840 |
| 8796.16 | 9356.8 | 9472.768 | 10062.72 | 9895.68 | 10512 | 10555.392 | 11212.8 | 10995.2 | 11680 |
| 17592.32 | 18713.6 | 18945.536 | 20125.44 | 19791.36 | 21024 | 21110.784 | 22425.6 | 21990.4 | 23360 |
| 35184.64 | 37427.2 | 37891.072 | 40250.88 | 39582.72 | 42048 | 42221.568 | 44851.2 | 43980.8 | 46720 |
| 70369.28 | 74854.4 | 75782.144 | 80501.76 | 79165.44 | 84096 | 84443.136 | 89702.4 | 87961.6 | 93440 |
| 140738.56 | 149708.8 | 151564.299 | 161003.52 | 158330.88 | 168192 | 168886.27 | 179404.8 | 175923.2 | 186880 |
| 281477.12 | 299417.6 | 303128.576 | 322007.04 | 316661.76 | 336384 | 337772.54 | 358809.6 | 351846.4 | 373760 |

Fig. 6a

EP 2 944 350 B1

| Dünndarm A. | Dünndarm E. | Blase A. | Blase E. | Niere A. | Niere E. | Kreislauf A. | Kreislauf E. | 3E A. | 3E E. |
|---|---|---|---|---|---|---|---|---|---|
| 0.0447363 | 0.0475259 | 0.0469766 | 0.0499023 | 0.05033203 | 0.0534668 | 0.0536875 | 0.05703125 | 0.0559243 | 0.0594072 |
| 0.0894727 | 0.0950518 | 0.0939531 | 0.0998047 | 0.10066406 | 0.1069336 | 0.107375 | 0.1140625 | 0.1118486 | 0.1188145 |
| 0.1789453 | 0.1901035 | 0.1879063 | 0.1996094 | 0.20132813 | 0.2138672 | 0.21475 | 0.228125 | 0.2236973 | 0.2376289 |
| 0.3578906 | 0.380207 | 0.3758125 | 0.3992188 | 0.40265625 | 0.4277344 | 0.4295 | 0.45625 | 0.4473945 | 0.4752578 |
| 0.7157813 | 0.7604141 | 0.751625 | 0.7984375 | 0.8053125 | 0.8554688 | 0.859 | 0.9125 | 0.8947891 | 0.9505156 |
| 1.4315625 | 1.5208281 | 1.50325 | 1.596875 | 1.610625 | 1.7109375 | 1.718 | 1.875 | 1.7895781 | 1.9010313 |
| 2.863125 | 3.0416563 | 3.0065 | 3.19375 | 3.22125 | 3.421875 | 3.436 | 3.65 | 3.5791579 | 3.8020625 |
| 5.72625 | 6.0833125 | 6.013 | 6.3875 | 6.4425 | 6.84375 | 6.872 | 7.3 | 7.1583125 | 7.604125 |
| 11.4525 | 12.166625 | 12.026 | 12.775 | 12.885 | 13.6875 | 13.744 | 14.6 | 14.316625 | 15.20825 |
| 22.905 | 24.33325 | 24.052 | 25.55 | 25.77 | 27.375 | 27.488 | 29.2 | 28.63325 | 30.4165 |
| 45.81 | 48.6665 | 48.104 | 51.1 | 51.54 | 54.75 | 54.976 | 58.4 | 57.2665 | 60.833 |
| 91.62 | 97.333 | 96.208 | 102.2 | 103.08 | 109.5 | 109.952 | 116.8 | 114.533 | 121.666 |
| 183.24 | 194.666 | 192.416 | 204.4 | 206.16 | 219 | 219.904 | 233.6 | 229.066 | 243.332 |
| 366.48 | 389.332 | 384.832 | 408.8 | 412.32 | 438 | 439.808 | 467.2 | 458.132 | 486.664 |
| 732.96 | 778.664 | 769.664 | 817.6 | 824.64 | 876 | 879.616 | 934.4 | 916.264 | 973.328 |
| 1465.92 | 1557.328 | 1539.328 | 1635.2 | 1649.28 | 1752 | 1759.232 | 1868.8 | 1832.528 | 1946.656 |
| 2931.84 | 3114.656 | 3078.656 | 3270.4 | 3298.56 | 3504 | 3518.464 | 3737.6 | 3665.056 | 3893.312 |
| 5863.68 | 6229.312 | 6157.312 | 6540.8 | 6597.12 | 7008 | 7036.928 | 7475.2 | 7330.112 | 7786.624 |
| 11727.36 | 12458.624 | 12314.624 | 13081.6 | 13194.24 | 14016 | 14073.856 | 14950.4 | 14660.224 | 15573.248 |
| 23454.72 | 24917.248 | 24629.248 | 26163.2 | 26388.48 | 28032 | 28147.712 | 29900.8 | 29320.448 | 31146.496 |
| 46909.44 | 49834.496 | 49258.496 | 52326.4 | 52776.96 | 56064 | 56295.424 | 59801.6 | 58640.896 | 62292.992 |
| 93818.88 | 99668.992 | 98516.992 | 104652.8 | 105553.92 | 112128 | 112590.848 | 119603.2 | 117281.79 | 124585.98 |
| 187637.76 | 199337.98 | 197033.98 | 209305.6 | 211107.84 | 224256 | 225181.696 | 239206.4 | 234563.58 | 249171.97 |
| 375275.52 | 398675.97 | 394067.97 | 418611.2 | 422215.68 | 448512 | 450363.392 | 478412.8 | 469127.17 | 498343.94 |

Fig. 6b

| Gbl A. | Gbl E. | Leber A. | Leber E. |
|---|---|---|---|
| 0.060398438 | 0.0641602 | 0.0623154 | 0.0661968 |
| 0.120796875 | 0.1283203 | 0.1246309 | 0.1323936 |
| 0.24159375 | 0.2566406 | 0.2492617 | 0.2647871 |
| 0.48318875 | 0.5132813 | 0.4985234 | 0.5295742 |
| 0.966375 | 1.0265625 | 0.9970469 | 1.0591484 |
| 1.93275 | 2.053125 | 1.9940938 | 2.1182969 |
| 3.8655 | 4.10625 | 3.9881875 | 4.2365938 |
| 7.731 | 8.2125 | 7.976375 | 8.4731875 |
| 15.462 | 16.425 | 15.95275 | 16.946375 |
| 30.924 | 32.85 | 31.9055 | 33.89275 |
| 61.848 | 65.7 | 63.811 | 67.7855 |
| 123.696 | 131.4 | 127.622 | 135.571 |
| 247.392 | 262.8 | 255.244 | 271.142 |
| 494.784 | 525.6 | 510.488 | 542.284 |
| 989.568 | 1051.2 | 1020.976 | 1084.568 |
| 1979.136 | 2102.4 | 2041.952 | 2169.136 |
| 3958.272 | 4204.8 | 4083.904 | 4338.272 |
| 7916.544 | 8409.6 | 8167.808 | 8676.544 |
| 15833.088 | 16819.2 | 16335.616 | 17353.088 |
| 31666.176 | 33638.4 | 32671.232 | 34706.176 |
| 63332.352 | 67276.8 | 65342.464 | 69412.352 |
| 126664.704 | 134553.6 | 130684.93 | 138824.7 |
| 253328.408 | 269107.2 | 261369.86 | 277649.41 |
| 506658.816 | 538214.4 | 522739.71 | 555298.82 |

Fig. 6c

**Fig. 7**

Fig. 8a

Fig. 8b

Fig. 8c

Fig. 8d

Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 202010010700 U1
- US 20070156178 A1
- EP 1529550 A1
- US 20050090867 A
- US 20080215114 A1
- WO 2006024150 A1
- DE 29709094 U1